# EUROPEAN PATENT SPECIFICATION

(11) **EP 1 326 960 B1**
(45) Date of publication and mention of the grant of the patent: **08.12.2004**
(21) Application number: 01979646.5
(22) Date of filing: 11.10.2001
(51) Int. Cl.: C12N 1/36, A61K 35/74

(54) **MICROBES HAVING AN ATTENUATING MUTATION COMPRISING A TRANSCRIPTION TERMINATOR**
MIKROBEN MIT EINER TRANSKRIPTIONSABBRECHER ENTHALTENDER DÄMPFENDER MUTATION
MICROBES A MUTATION D'ATTENUATION RENFERMANT UN TERMINATEUR DE TRANSCRIPTION

(30) Priority: 12.10.2000 US 689123
(43) Date of publication of application: 16.07.2003
(73) Proprietor: Washington University in St. Louis, St. Louis, MO 63130 (US); Megan Health, Inc., St. Louis, MO 63110 (US); Curtiss, Roy III, St. Louis, MO 63112 (US); Tinge, Steven A., Lebanon, IL 62254 (US)
(72) Inventor: CURTISS, Roy, III, St. Louis, MO 63112 (US); TINGE, Steven, A., Lebanon, IL 62254 (US)
(74) Representative: Baldock, Sharon Claire
(86) International application number: PCT/US2001/031606
(87) International publication number: WO 2002/030457

(56) References cited:
- WO-A-98/02530
- WO-A-99/45120

### SEQUENCE LISTING

<110> WASHINGTON UNIVERSITY IN ST. LOUIS MEGAN HEALTH, INC.
<120> MICROBES HAVING AN ATTENUATING MUTATION COMPRISING A TRANSCRIPTION TERMINATOR
<130> 15060-5
<140>
   <141>
<160> 25
<170> PatentIn Ver. 2.1
<210> 1
   <211> 480
   <212> DNA
   <213> Escherichia coli
<400> 1
   agattaaatc agaacgcaga agcggtctga taaaacagaa tttgcctggc ggcagtagcg 60
   cggtggtccc acctgacccc atgccgaact cagaagtgaa acgccgtagc gccgatggta 120
   gtgtggggtc tccccatgcg agagtaggga actgccaggc atcaaataaa acgaaaggct 180
   cagtcgaaag actgggcctt tcgttttatc tgttgtttgt cggtgaacgc tctcctgagt 240
   aggacaaatc cgccgggagc ggatttgaac gttgcgaagc aacggcccgg agggtggcgg 300
   gcaggacgcc cgccataaac tgccaggcat caaattaagc agaaggccat cctgacggat 360
   ggcctttttg cgtttctaca aactcttcct gtcgtcatat ctacaagcca tccccccaca 420
   gatacggtaa actagcctcg tttttgcatc aggaaagcag ctgttttggc ggatgagaga 480
<210> 2
   <211> 59
   <212> RNA
   <213> Escherichia coli
<400> 2
   caucaaauaa aacgaaaggc ucagucgaaa gacugggccu uucguuuuau cuguuguuu 59
<210> 3
   <211> 35
   <212> RNA
   <213> Escherichia coli
<400> 3
   guaggacaaa uccgccggga gcggauuuga acguu 35
<210> 4
   <211> 46
   <212> RNA
   <213> Escherichia coli
<400> 4
   aaauuaagca gaaggccauc cugacggaug gccuuuuugc guuucu 46
<210> 5
   <211> 41
   <212> DNA
   <213> Escherichia coli
<400> 5
   gaatttagcc cgcctaatga gcgggctttt ttttgtgatc c 41
<210> 6
   <211> 34
   <212> RNA
   <213> Escherichia coli
<400> 6
   gaauuuagcc cgccuaauga gcgggcuuuu uuuu 34
<210> 7
   <211> 34
   <212> RNA
   <213> Escherichia coli
<400> 7
   aaaaaaaagc ccgcucauua ggcgggcuaa auuc 34
<210> 8
   <211> 60
   <212> DNA
   <213> Escherichia coli
<400> 8
   attatattac taattaattg gggaccctag aggtcccctt ttttatttta aaaatttttt 60
<210> 9
   <211> 44
   <212> RNA
   <213> Escherichia coli
<400> 9
   uaauuaauug gggacccuag agguccccuu uuuuauuuua aaaa 44
<210> 10
   <211> 43
   <212> RNA
   <213> Escherichia coli
<400> 10
   uuuuuaaaau aaaaagggga ccucuagggu ccccaauuaa uua 43
<210> 11
   <211> 71
   <212> DNA
   <213> Escherichia coli
<400> 11
   taaataaaag cagtttacaa ctcctagaat tgtgaatata ttatcacaat tctaggatag 60
   aataataaaa a 71
<210> 12
   <211> 57
   <212> RNA
   <213> Escherichia coli
<400> 12
   guuuacaacu ccuagaauug ugaauauauu aucacaauuc uaggauagaa uaauaaa 57
<210> 13
   <211> 57
   <212> RNA
   <213> Escherichia coli
<400> 13
   uuuauuauuc uauccuagaa uugugaauau auuaucacaa uucuaggagu uguaaac 57
<210> 14
   <211> 57
   <212> DNA
   <213> Escherichia coli
<400> 14
   aaattatgcg aaaggccatc ctgacggatg gcctttttgc attggcgcag aaaaaaa 57
<210> 15
   <211> 45
   <212> RNA
   <213> Escherichia coli
<400> 15
   aaauuaugcg aaaggccauc cugacggaug gccuuuuugc auugg 45
<210> 16
   <211> 45
   <212> RNA
   <213> Escherichia coli
<400> 16
   ccaaugcaaa aaggccaucc gucaggaugg ccuuucgcau aauuu 45
<210> 17
   <211> 41
   <212> DNA
   <213> Escherichia coli
<400> 17
   tcgacgaact taaataatgc ctgcctcacc ctcttttctt a 41
<210> 18
   <211> 26
   <212> DNA
   <213> Artificial Sequence
<220>
   <223> Description of Artificial Sequence: Primer
<400> 18
   gatctaagaa aagagggtga ggcagg 26
<210> 19
   <211> 39
   <212> DNA
   <213> Artificial Sequence
<220>
   <223> Description of Artificial Sequence: trpA transcription terminator
<400> 19
   gatcgcggcc gcccgcctaa tgagcgggct ttttttgcc 39
<210> 20
   <211> 39
   <212> DNA
   <213> Artificial Sequence
<220>
   <223> Description of Artificial Sequence: trpA transcription terminator
<400> 20
   aattggcaaa aaaagcccgc tcattaggcg ggcggccgc 39
<210> 21
   <211> 14
   <212> DNA
   <213> Artificial Sequence
<220>
   <223> Description of Artificial Sequence: phoQ coding sequence
<400> 21
   aattcatgaa taaa 14
<210> 22
   <211> 10
   <212> DNA
   <213> Artificial Sequence
<220>
   <223> Description of Artificial Sequence: phoQ coding sequence
<400> 22
   tttattcatg 10
<210> 23
   <211> 8
   <212> DNA
   <213> Artificial Sequence
<220>
   <223> Description of Artificial Sequence: Primer
<400> 23
   ggcgcgcc 8
<210> 24
   <211> 36
   <212> DNA
   <213> Artificial Sequence
<220>
   <223> Description of Artificial Sequence: Primer
<400> 24
   ttggcgcgcc tgaataaatt tgctcgccat tttctg                    36
<210> 25
   <211> 32
   <212> DNA
   <213> Artificial Sequence
<220>
   <223> Description of Artificial Sequence: Primer
<400> 25
   ttggcgcgcc taaaccagac aaatagtcac cc                        32

## Description

### BACKGROUND OF THE INVENTION

### (1) Field of the Invention

This invention relates generally to attenuated microbes and, more particularly, to microbes having an attenuating chromosomal mutation comprising a recombinant transcription factor for use in vaccines and delivery vehicles for genes and gene products and to methods therefor.

### (2) Description of the Related Art

The use of vaccines has proven to be an effective approach for controlling infectious diseases. Of the different types of vaccines, live attenuated microorganisms have been the most successful and offer the most promising candidates for future oral vaccines and delivery systems for heterologous antigens. (for reviews see Bumann et al., *FEMS Immunol*. *Med*. *Microbiol. 27*:357-364, 1999; Liljeqvist et al., *J*. *Biotechnol 73*:1-33, 1999).

Ideally, live attenuated vaccines must achieve a balance between attenuation and immunogenicity so as to elicit an immune response without producing disease symptomatology or other side effects (Curtiss in *New Generation Vaccines*, Woodrow and Levine, Eds., Marcel Dekker, Inc., New York, 1990, pp. 161-168; Dorner et al., *Ann Med 31*:51-60,1999). Strategies for producing attenuated strains typically involve mutagenesis of biosynthetic genes, regulatory genes and/or genes involved in virulence (Dogget and Brown, in *Mucosal Vaccines,* Kiyono et al., Eds., Academic Press, San Diego, 1996, pp. 105-118). The mutagenic changes elicited include gene insertions, deletions, base changes, inversions, translocations, duplications and the like. However, these mutagenic changes can produces results other than complete abolition of the function encoded by the damaged gene. For example, a point mutation can produce an aberrant protein which can be partially functional. Furthermore, insertions can produce fusion proteins having unpredictable and sometimes toxic effects on the microbe. Mutagenesis can also result in alterations not only in a target gene but also in neighbouring genes. For example, the mutant *galE* derivative, Ty21a of *S*. *typhi* Ty2 was generated by chemical mutagenesis which produced mutations in genes in addition to the *galE* gene. These mutations resulted in hyperattenuation of the microbe and low vaccine efficacy for the Ty21a strain (Forrest, in C.R.C. Press, Inc., 1994, pp. 59-80). Another example of the unpredictability of mutagenesis has been reported for the transposon-induced deletion mutation of the *crp* gene. In some instances this was found to produce an attenuation which could not be complemented by the wild-type *crp* gene to restore wild-type virulence. Such deletion mutations were discovered to extend into adjacent genes to render the *Salmonella* less able to colonize deep tissues without impairing the ability to colonize the gut associated lymphoid tissue (see U.S. Patent No. 5,387,744).

One approach to minimize the unpredictability of mutagenesis has emerged in the development techniques that produce defined deletions. (see for example, Ling et al., *Anal. Biochem. 254*:157-178, 1997; U.S. Patent No. 6,024,961, Examples 2 and 3). Nevertheless, defined deletions like other deletion mutations, can potentially produce undesirable changes in the function encoded by the damaged gene. For example, a defined deletion mutation can produce an aberrant protein product that is either truncated or which has an abnormal C-terminal amino acid sequence due to a frame shift caused by the deletion. Furthermore, deletion of the entire coding sequence of a gene which leaves the promoter intact could potentially result in the bacterial cell compensating by enhancing RNA polymerase recognition of the promoter and transcription through the deleted gene to include the adjacent gene. If the adjacent gene is transcribed in the opposite orientation, then mRNA synthesized would be an anti-sense mRNA that would interfere with or prevent the normal expression of that adjacent gene. The production of an aberrant protein product or either the over-expression or the inhibition of a gene adjacent to the deletion could modify attenuation or immunogenicity.

Further problems can arise in situations in which a promoter is included in an insertion sequence. This can occur, for example where the inserted sequence is designed to contain a heterologous reporter gene or some other gene, for expression independent of the expression of the mutant gene (see for example, Sousa et al., *Microbiol*. *143*:2071-2078, 1997; Vagner et al., *Microbiol. 144*:3097-3104, 1998). In some instances, the inserted promoter can potentially cause an expression of adjacent genes to produce unpredictable effects on attenuation and immunogenicity of the microbe. Thus, there remains a continuing need for new approaches for constructing vaccine strains which produce discrete effects on specific gene targets and which, as a result, produce predictable effects on attenuation and immunogenicity of the microbe.

In recent years, transcription termination has emerged as an important site for the regulation of gene expression (for reviews see Henkin, *Current Opin. Microbiol. 3*:149-153, 2000; Henkin, *Ann. Rev. Genet. 30*:35-57, 1996). Transcription termination occurs when the RNA polymerase encounters a termination signal, stops adding nucleotides to the newly synthesized RNA, separates the DNA-RNA hybrid, releases the newly synthesized transcript and dissociates from the bNA template (for reviews see Mooney et al., *J. Bacteriol. 180*:3265-3275, 1998; Henkin, *supra*; Weisberg et al., *J*. *Bacteriol. 181*:359-367, 1999). At least three types of termination signals are used by bacteria: (1) intrinsic terminators or Rho-independent terminators which are made up of a G-C-rich stem loop portion followed by a series of U nucleotides (for review see Record et al., in *Escherichia coli and Salmonella: Cellular and Molecular Biology*, *2nd ed*., Neidhardt et al., eds., ASM Press, Washington, D.C., 792-821, 1996), (2) Rho-dependent terminators which act through a binding site for the Rho protein on the nascent transcript (for review see Richardson, *J*. *Biol*. *Chem*. *271*:1251-1254, 1996) and (3) persistent RNA-DNA hybrid terminators at which pairing of nascent RNA to the template just upstream from the transcription elongation complex dissociates a complex containing 3'-proximal U-rich RNA (Tomizawa et al, *Cell* 51:623-630, 1987). Although transcription terminators are known to regulate gene expression and some insertion cassettes have incorporated transcription terminators into bacterial chromosomes, transcription terminators have not been heretofore used to produce attenuated microbes for use in vaccine preparations.

One group has reported on a naturally occurring insertion of transcription terminators in *Yersinia pestis* which prevents the expression of a functional *inv* gene product (Simonet et al., *Infect. Immun*. *64*:375-379, 1996; Odaert et al., *J*. *Bacteriol*. *180*:178-181). Thus, although other *Yersinia* species penetrate eukaryotic cells through invasin, *Yersinia pestis,* which is the causative agent for plague, is unable to do so as a result of multiple insertions of a transcription terminator in the *inv* gene. The wild-type microbe, however, is not suitable for use as an attenuated vaccine strain inasmuch as *Yersinia pestis* is highly virulent even in absence of a functional *inv* gene product.

Other groups have reported on the use of transcription terminators as part of a chromosomal insertion cassette. For example, Reeves et al. (*J*. *Bacteriol. 181*:7098-7106, 1999) studied the transcriptional organization of the erythromycin biosynthetic *ery* gene cluster of *S. erythraea* by insertional inactivation of erythromycin biosynthetic genes using mutants constructed to contain an *rrn* terminator at specific sites. Nevertheless, this group did not construct vaccine strains nor did the microbes produced by this group have attenuating mutations comprising a transcription terminator.

Reeve et al. (*Microbiol. 145*:1307-1316, 1999) reported on the development of cassettes containing an antibiotic resistance marker with the *tac* promoter and *trpA* terminator for use in monitoring transcriptional activity in different genetic backgrounds in root nodule bacteria. Nevertheless, this group did not disclose or suggest the use of the cassette in producing an attenuating mutation in a microbe nor did they suggest the use of the cassette in producing a vaccine strain.

Curtiss et al. (WO1996/US/09774) reported on the production of microbes having an environmentally limited viability system. The disclosed mutant microorganisms were constructed with a deletion in the chromosomal *asd* gene and the insertion of a cassette containing promoters and transcription terminators in the gene site. The microbes ultimately constructed also contained a plasmid with an *asd* gene (see for example, WO1996/US/09774, Figures 4, 8, 11, and 13). This *asd* gene in the plasmid complemented the deleted chromosomal *asd* gene so that the microbes were not attenuated by the chromosomal *asd* gene mutation. Thus, this publication did not disclose or suggest the construction of an attenuating chromosomal *asd* gene mutation containing a transcription terminator nor did this publication suggest the use of transcription terminators in general as an attenuating strategy for the preparation of vaccine strains.

Thus, earlier work with transcription terminators did not report on the use of transcription terminators to attenuate microbes or as part of an attenuation strategy to produce new vaccine preparations. Hence, notwithstanding the use of transcription terminators in the study of gene structure and function, there remains a continuing need for new approaches for attenuating microbes and for producing vaccines in a manner that both attenuates and maintains the immunogenicity of the microbe.

### BRIEF DESCRIPTION OF THE INVENTION

Accordingly, the inventors herein have succeeded in discovering that transcription terminators can be inserted into bacterial genes in a novel approach for attenuating the bacteria. The transcription terminator sequence, which is inserted in the gene or gene site, produces a transcription termination so that the coding sequence of the gene is not fully transcribed and a functional gene product is not expressed. Such an insertion in an operon can prevent transcription and expression of genes downstream from the insertion. The approach has applicability in the construction of attenuated microbes which can be used in vaccines and as carrier microbes for the expression of a desired gene product.

Thus, in one embodiment the present invention is directed to a composition comprising a microbe which has an attenuating mutation comprising a sequence which includes a transcription terminator inserted into a chromosomal gene or gene site. By gene site, it is meant that in constructions in which a target gene is deleted in part or in whole, a transcription terminator is inserted into the chromosomal position that would have been occupied by the gene had it not been deleted. The composition is in a pharmaceutically acceptable preparation. The sequence which includes the transcription terminator insertion can in some instances also include a recombinant promoter. The promoter may be present for expression of a recombinant gene which is also included in the insertion sequence. The expression of the recombinant gene is thus independent of the promoter of the gene into which the promoter, recombinant gene and transcription terminator are inserted. In these instances, the transcription terminator serves to prevent the inserted promoter from altering expression of genes neighbouring the mutant gene. Alternatively, the transcription terminator can be inserted in absence of insertion of a recombinant promoter.

Any of a number of genes can be targeted for insertion of the transcription terminator sequence for attenuation of the microbe. In particular, such genes can be biosynthetic genes, regulatory genes and/or genes involved in virulence.

In a particularly preferred aspect of this embodiment, the microbe further contains a recombinant gene encoding for expression, a desired gene product from a pathogen such as a virus, bacterium, protozoan, parasite or fungus. The recombinant gene can also encode a product capable of suppressing modulating or augmenting an immune response in the individual. Moreover, the recombinant gene can encode an autoantigen such as a gamete specific antigen or an allergen to the individual.

In another embodiment, the present invention is directed to a vaccine. The vaccine comprises a microbe having an attenuating insertion which comprises a recombinant transcription terminator inserted in a chromosomal gene, in a pharmaceutically acceptable preparation. In one aspect of this embodiment, the vaccine microbe can further contain a recombinant gene encoding for expression, a desired gene product which comprises an epitope from a pathogen such as a virus, bacterium, protozoan, parasite or fungus. The recombinant gene can also encode an autoantigen such as a gamete specific antigen or an allergen to the individual.

Another embodiment of the present invention is directed to a method of immunizing an individual against a pathogen. The method comprises administering to the individual a composition comprising a derivative of a pathogenic microbe. By derivative, it is meant that the pathogenic microbe has been altered to attenuate pathogenicity by insertion of a recombinant transcription terminator in a chromosomal gene. In one aspect of this embodiment, the microbe can further comprise a recombinant gene which encodes an epitope from the pathogen. The pathogen can be a virus, bacterium, protozoan, parasite or fungus.

The present invention, in another embodiment, is also directed to a method for producing a carrier microbe for delivery of a desired gene product to an individual. The method comprises generating an attenuated derivative of a pathogenic microbe having a recombinant gene encoding the desired gene product and an attenuating mutation comprising a transcription terminator inserted in a chromosomal gene.

The present invention in another embodiment is directed to a method for delivering a desired gene product to an individual. The method comprises administering to the individual a composition comprising a microbe having an attenuating mutation comprising a recombinant transcription terminator inserted in a chromosomal gene. The microbe can further comprise a recombinant gene encoding the desired gene product. The gene product encoded by the recombinant gene can be from a pathogen such as a virus, bacterium, protozoan, parasite or fungus. The recombinant gene can also encode a product capable of suppressing, modulating or augmenting an immune response in the individual. Moreover, the recombinant gene can encode an autoantigen such as a gamete specific antigen or an allergen to the individual.

In each of the above embodiments, the transcription terminator can be a Rho-dependent or, preferably, a Rho-independent terminator including but not limited to *rrnB* 5s rRNA T1T2, *trpA*, T4 gene 32, T4 *ipIII* gene, or *rrfG* 5S rRNA; the genes into which the transcription terminator can be inserted to attenuate the microbe include, but are not limited to, a *pab* gene, a *pur* gene, an *aro* gene, *asd*, a *dap* gene, *nadA*, *pncB*, *galE*, *pmi*, *fur*, *rpsL*, *ompR*, *htrA*, *hemA*, *cdt, cya*, *crp*, *phoP*, *phoQ*, *rfc*, *poxR*, or *galU*; and the attenuated microbes are preferably enteropathogenic microbes species including, but not limited to, *Salmonella, Shigella, Escherichia* or hybrid thereof. In addition, the insertion of the transcription terminator can be into a gene which has a defined deletion which includes all or part of the coding region of said gene, the promoter region of said gene or both the coding region and promoter region of said gene. Specific examples of microbes within the present invention are MGN-1362, χ8298 and χ8429.

Among the several advantages achieved by the present invention, therefore, may be noted the provision of microbes having a discrete attenuating mutation which produces a predictable attenuation and immunogenicity of the microbe; the provision of microbes with attenuating mutations in target genes in which the mutations do not effect neighbouring genes thereby avoiding unexpected side effects from the mutation; the provision of vaccine compositions, delivery vehicles and methods for beneficially administering these compositions and delivery vehicles for the immunization or for administering a desired gene product; and the provision of methods for producing the attenuated microbes.

### BRIEF DESCRIPTION OF THE DRAWINGS

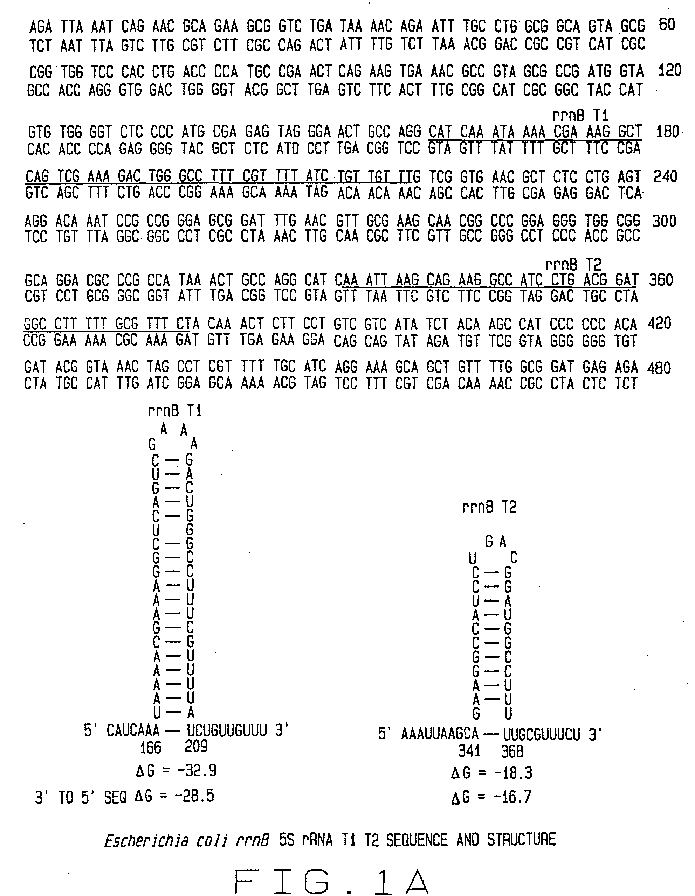

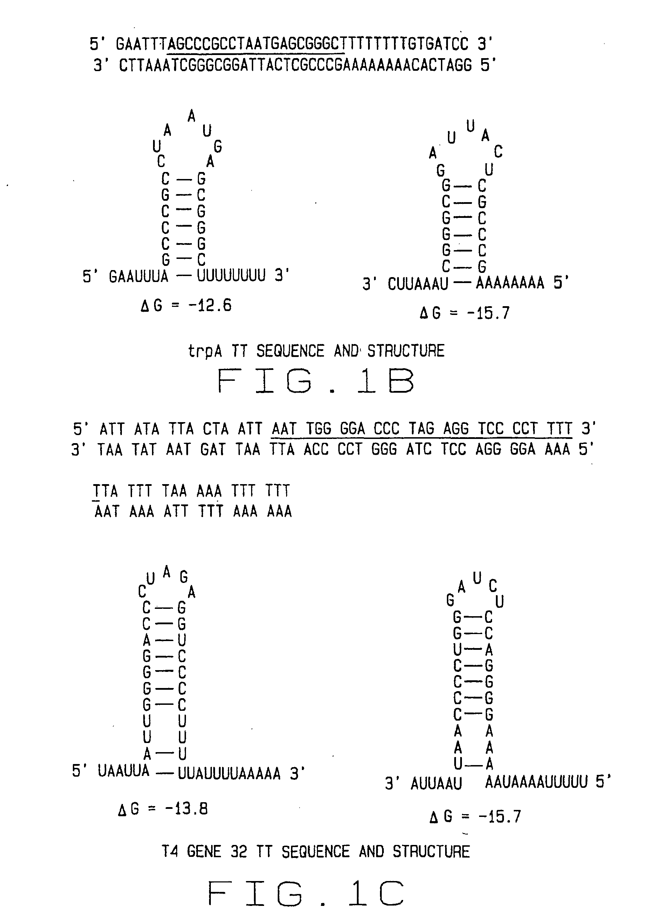

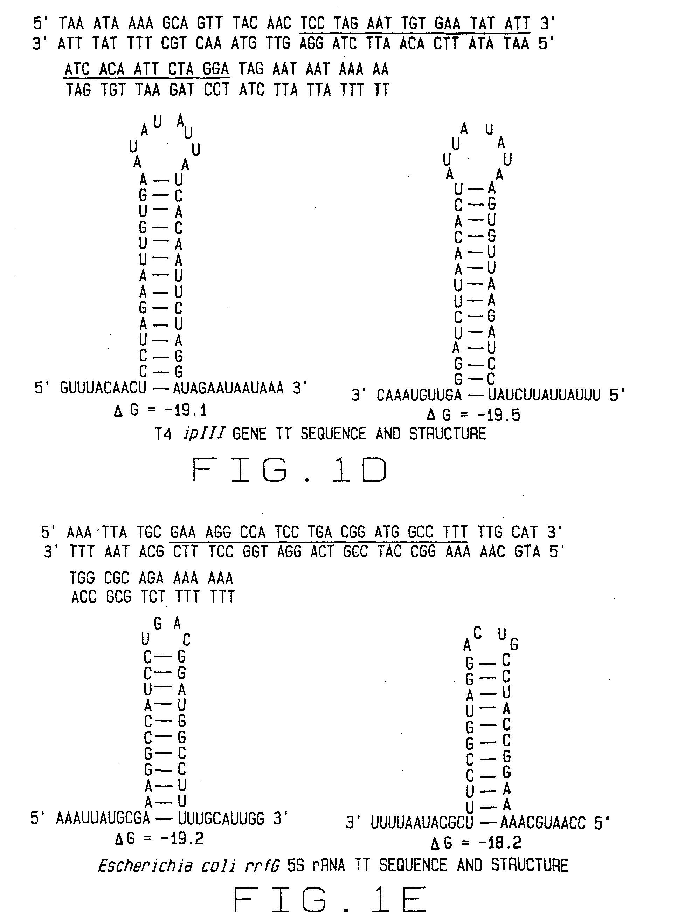

Figure 1 illustrates the sequences of transcription terminators (A) *E. coli rrnB* 5SrRNA T1 T2 (SEQ ID NO:1) along with the RNA stem loop structure of *rrnB* T1 corresponding to nucleotides 160-218 (SEQ ID NO:2), a second RNA stem loop structure between *rrnB* T1 and *rrnB* T2 corresponding to nucleotides 238-273 (SEQ ID NO:3) and the RNA stem loop structure of *rrnB* T2 corresponding to nucleotides 332-377 (SEQ ID NO:4); (B) *trpA* TT (SEQ ID NO:5) along with RNA stem loop structures of the each of the corresponding complementary single stranded sequences (SEQ ID NO:6 and SEQ ID NO:7); (C) T4 gene 32 TT (SEQ ID NO:8) along with the RNA stem loop structures of each of the complementary single stranded sequences corresponding to nucleotides 11-54 (SEQ ID NO:9 and SEQ ID NO:10); (D) T4 *ipIII* gene TT (SEQ ID NO:11) along with the RNA stem loop structures of each of the complementary single stranded sequences corresponding to nucleotides 13-69 (SEQ ID NO:12 and SEQ ID NO:13); and (E) *E.coli rrfG* 5S rRNA TT (SEQ ID NO:14) along with the RNA stem loop structures of each of the complementary single stranded sequences corresponding to nucleotide 1-45 (SEQ ID NO:15 and SEQ ID NO:16).
Figure 2 illustrates pMEG-443.
Figure 3 illustrates pMEG-105.
Figure 4 illustrates pMEG-096.
Figure 5 illustrates pMEG-611.
Figure 6 illustrates the construction of *ΔphoPQ23*.
Figure 7 illustrates pMEG-685.
Figure 8 illustrates pMEG-549.
Figure 9 illustrates pMEG-375.
Figure 10 illustrates pMEG-550.
Figure 11 illustrates the insertion of *trpA* terminator into *ΔphoP24* deletion showing a sequence which includes nucleotides 0-40 which are 5' to the coding sequence of the *phoP* gene (TCGACGAACTTAAATAATGCCTGCCTCACCCTCTTTTCTTA, SEQ ID NO:17), a sequence including the nucleotides 20-40 complementary thereto (GATCTAAGAAAAGAGGGTGAGGCAGG, SEQ ID NO:18), a sequence including
   the *trpA* transcription terminator (GATCGCGGCCGCCCGCCTAATGAGCGGGCTTTTTTTGCC, SEQ ID NO:19 and AATTGGCAAAAAAAGCCCGCTCATTAGGCGGGCGGCCGC, SEQ ID NO:20) and a portion of the phoQ coding sequence (AATTC ATG AAT AAA, SEQ ID NO:21 with its complement TTTATTCATG, SEQ ID NO:22) along with the first three encoded amino acids of the *phoQ* gene.

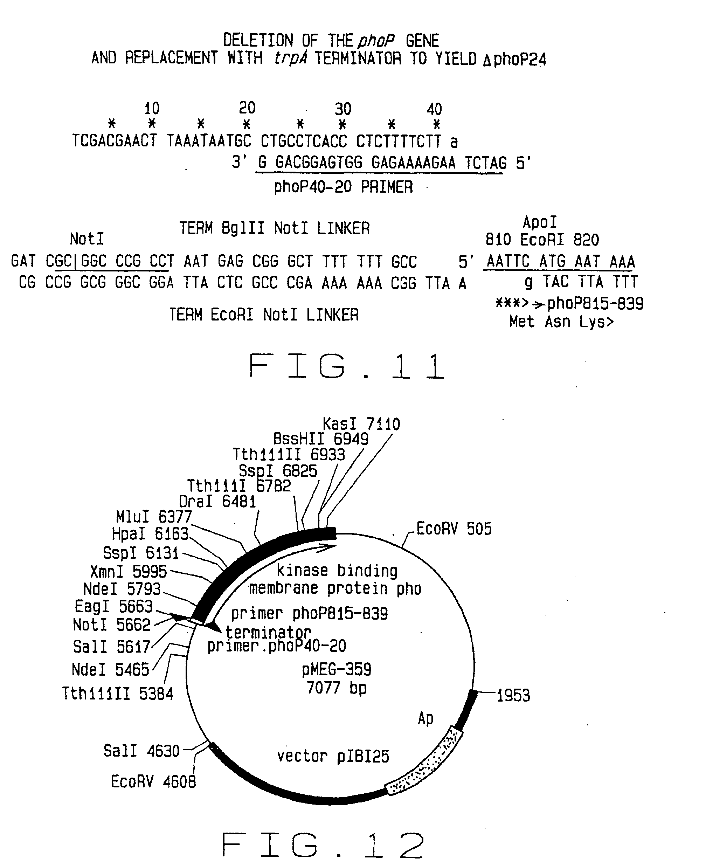

Figure 12 illustrates pMEG-359.

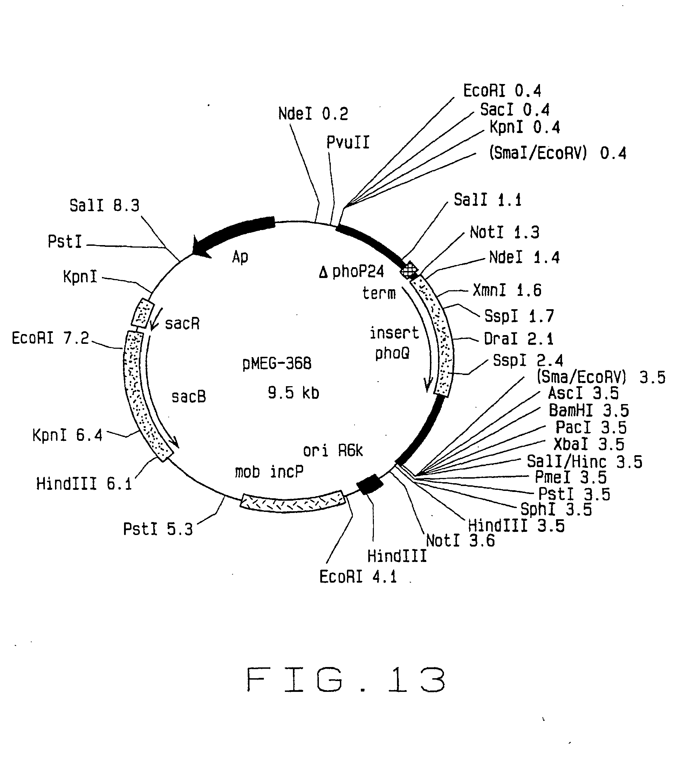

Figure 13 illustrates pMEG-368.

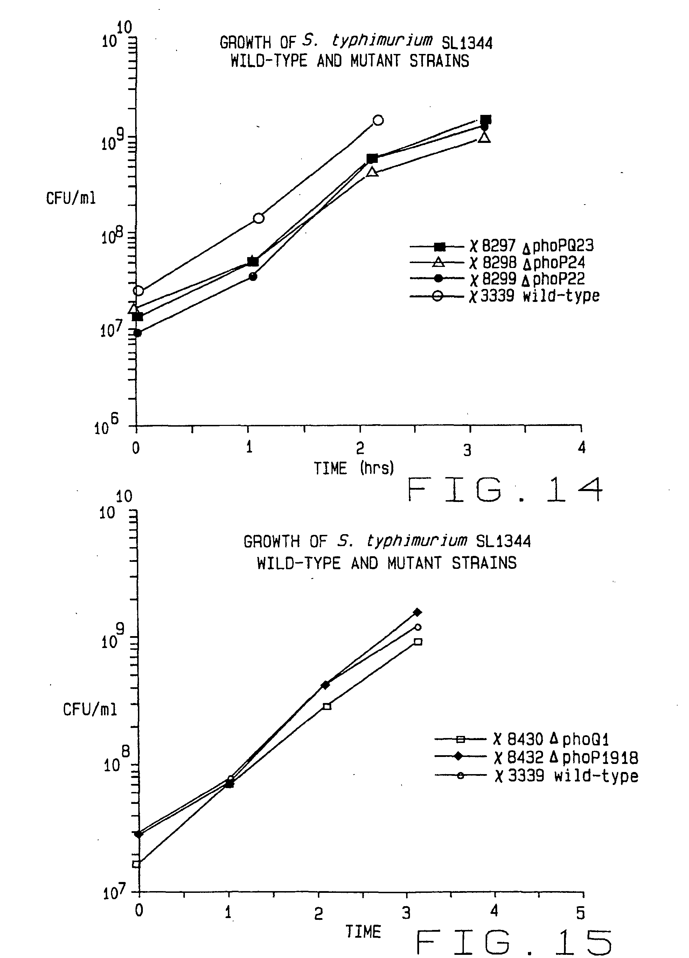

Figure 14 illustrates the growth curves of *S*. *typhimurium* SL1344 wild-type and mutant strains χ8297, χ8298, χ8299 and χ3339.
Figure 15 illustrates the growth curves of *S*. *typhimurium* SL1344 wild -type and mutant strains χ8430, χ8432 and χ3339.

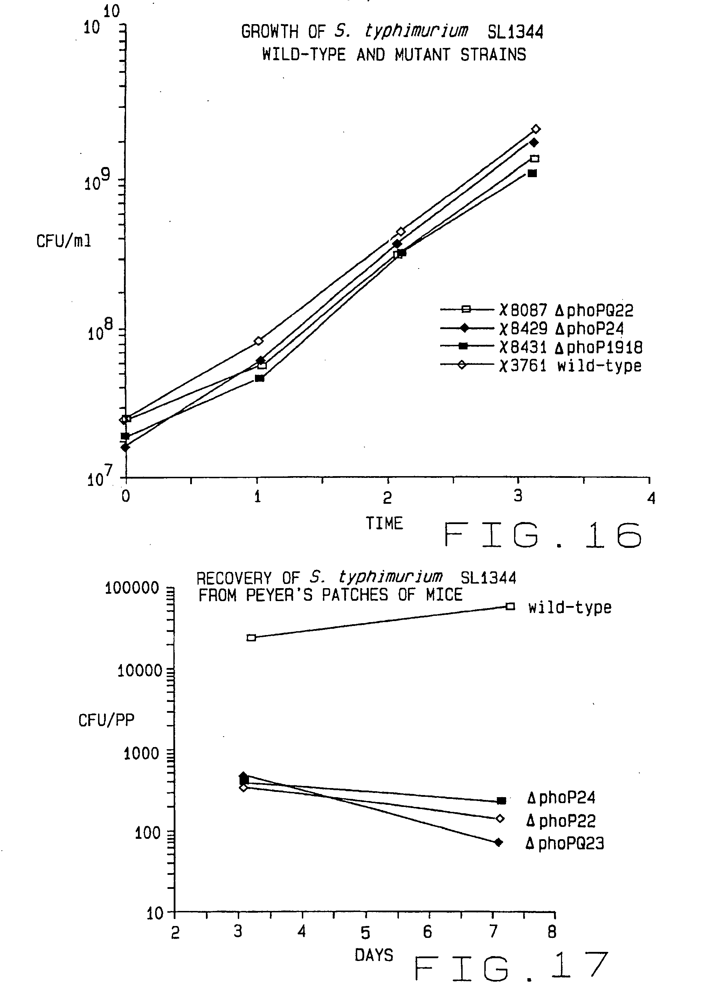

Figure 16 illustrates the growth curves of *S*. *typhimurium* UK-1 wild-type and mutant strains χ8087, χ8429, χ8431 and χ3761.
Figure 17 illustrates the colonization of Peyer's patches at days 3 and 7 by *S. typhimurium* SL1344 wild-type and mutant strains χ3339, χ8297, χ8298 and χ8299.

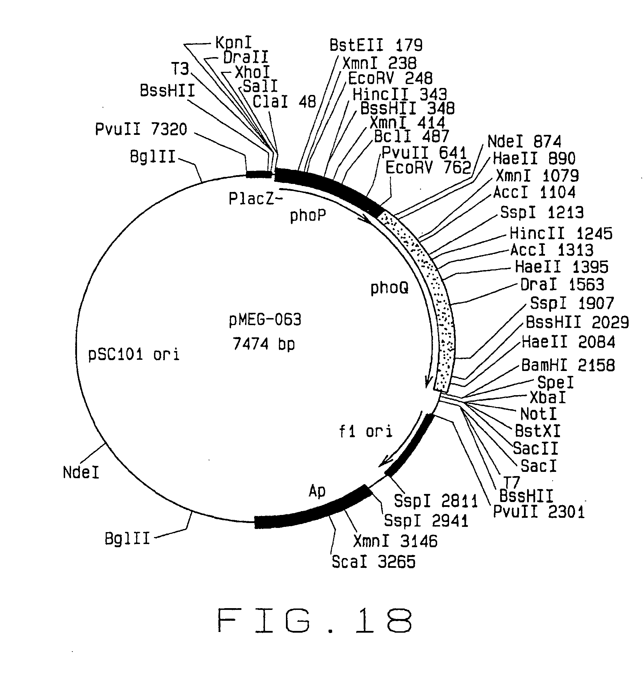

Figure 18 illustrates pMEG-063.

### DETAILED DESCRIPTION OF THE INVENTION

This invention is based upon the discovery that transcription terminators can be inserted in a bacterial gene in an attenuating strategy which not only attenuates the virulence of the bacteria, but also serves to restrict the effect of the attenuating mutation to the target gene or operon of the bacteria. This new approach not only avoids the often unpredictable actions of other attenuating strategies on adjacent genes which can be diminish the immunogenicity of the bacteria or otherwise deleteriously effect the bacteria, it also allows a concerted action on all of the genes of an operon. This is because the attenuating and/or restrictive action occurs functionally at the level of transcription and is not directly dependent upon changing the coding sequence of a gene as are other attenuating strategies.

Transcription termination regulates gene expression at the level of transcription by signaling the RNA polymerase to stop adding nucleotides to the newly synthesized transcript which then becomes released and subsequently dissociated from the DNA. The term "transcription terminator" as used herein can be operationally defined as a site along the template DNA at which the rate of release of an RNA transcript is greater than the rate of addition of the next nucleotide. At least three types of termination signals are used by bacteria: (1) intrinsic terminators or Rho-independent terminators (2) Rho-dependent terminators and (3) persistent RNA-DNA hybrid terminators (Mooney et al. 1998, *supra*). Such transcription terminators are included within the scope of the present invention. It is also intended that transcription terminators within the present invention include any transcription termination signaling sequence, whether or not such sequence normally occurs in wild-type microbes, so long as such sequence functions to preferentially halt RNA polymerase activity and cause release a nascent RNA transcript.

Intrinsic transcription terminators are characterized by about 20 bp of a G-C-rich sequence which forms a stem-loop or hairpin structure. This is immediately followed by a poly U region of at least 3 and, typically, about 7 to 9 U residues to the terminated RNA 3' end (For review see Richardson et al., in *Escherichia coli and Salmonella Cellular and Molecular Biology, Vol*. *I, 2d*, *ed.,* Neidhardt et al., eds., ASM Press, Washington, D.C., 1996, pp. 822-848). A large number of intrinsic, Rho-independent transcription terminators have been identified (see for example, Carafa et al., *J*. *Mol. Biol*. *216*:835-858, 1990; Brendel et al., *J*. *Biomol*. *Struct. Dynam.3*:705-723, 1986).

Rho-dependent transcription termination acts through the binding of the Rho protein to a Rho utilization site on the nascent transcript. Rho-dependent terminators consist of two separate but overlapping sequences which together extend over 150 to 200 bp of DNA (Richardson et al., *supra*). One sequence constitutes the transcription stop point region while the other sequence is upstream from this and is referred to as a Rho utilization site. The Rho utilization site is believed to represent the binding site for the Rho protein.(*Id*.; Richardson, *Biochim*. *Biophys*. *Acta 1048*:127-138, 1990). A number of Rho-dependent transcription terminators have been identified (see for example Brendel et al., *supra*; Zalatan et al, *J Biol Chem 268*:17051-17056, 1993).

Preferred transcription terminators are intrinsic, Rho-independent terminators including rrnB 5s rRNA T1T2, *trpA*, T4 gene 32, T4 *ipIII* gene, and *rrfG* 5S rRNA.

In some instances it can be desirable to use more than one transcription terminator. Because a transcription terminator need only preferentially release an RNA transcript over addition of the next nucleotide, some transcription terminators are considered weak transcription terminators in that RNA polymerase is capable of reading through the transcription terminator site. When using such transcription terminators, it can be desirable to incorporate more than one such terminator in series in the insertion cassette. In other instances, because transcription can sometimes occur in either direction from a particular promoter site, it can also sometimes be desirable to include at least one transcription terminator on either end of an insertion cassette.

A gene as described herein is intended to mean the full length nucleic acid sequence including the coding sequence which further includes any leader sequence and also including any and all regulatory sequences associated with that gene. Transcription terminators and cassettes including transcription terminators can be inserted at any site in a target gene.

In some instances it can be desirable to include the transcription terminator in a cassette containing a coding region such as for a reporter and a promoter. However, the promoter insertion can potentially cause transcription of adjacent sequence 3' to the target gene to produce unpredictable effects on the microbe. In such instances, a transcription terminator can be included in the cassette to prevent the promoter from eliciting expression of genes 3' to the insertion site. Such cassette insertions can contain, for example, a heterologous gene encoding a desired gene product. It is often desirable to have foreign antigens encoded by sequences on high copy number plasmid vectors so that a large amount of the foreign antigen is expressed and a greater immune response is elicited in a vaccinated individual. Nevertheless, it is sometimes preferable to insert a gene encoding a foreign antigen into the bacterial chromosome. For example, some foreign antigens represent appendages such as fimbriae that serve as adhesins enabling a pathogen to attach to a particular receptor on a particular cell type within an animal host. Immune responses against such fimbrial adhesins are effective when antibodies to the fimbrial adhesin block the ability of a microorganism having that fimbrial adhesin from attaching to a receptor on a given target cell. Thus protective immunity against adhesins serves as a very important first line of defense in precluding the ability of pathogens to attach to, invade, and/or colonize an animal host. However, fimbrial adhesins are complex structures and putting genes encoding them on high copy number vectors usually leads to toxicity and inability of the recombinant vaccine strain to grow well or even to colonize the animal host. As a consequence, such vaccine constructs are rather nonimmunogenic in inducing the desired immune response. In contrast, since fimbrial adhesins are highly immunogenic when they are synthesized by coding sequences located in the chromosome, a high level immune response is often the result.

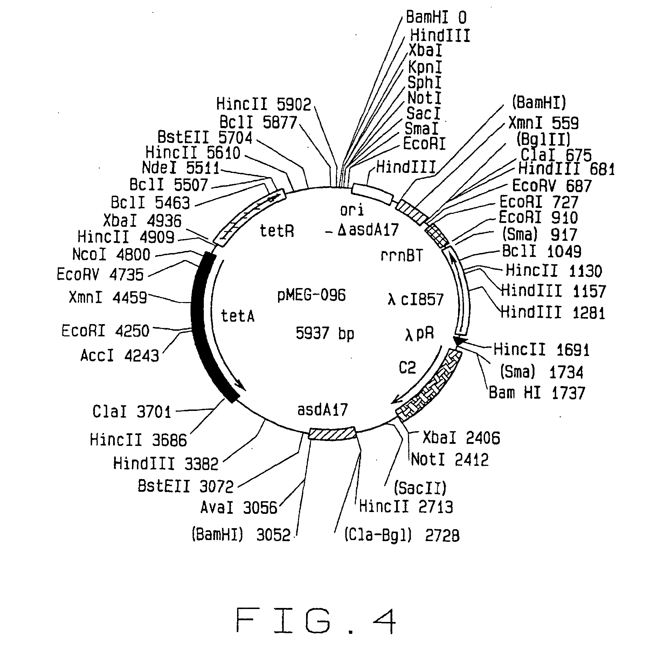

In other instances, the heterologous gene can encode a gene product which serves to regulate other genes within the microbe. Such a construct was disclosed in by Curtiss et al. (WO1996/US/09774) for use in generating microbes having an environmentally limited viability system. The disclosed mutant microorganisms were constructed with a deletion in the chromosomal *asd* gene and an insertion sequence into the chromosomal *asd* gene. The inserted sequence contained the coding sequence for regulatory genes which serve as activators or repressors of other genes specifying synthesis of essential gene products or lethal gene products encoded on a plasmid within the microbe. In such environmentally limited viability systems, the strain survives under permissive conditions by expression of essential genes and nonexpression of lethal genes, and under nonpermissive conditions, the strain ceases expression of essential genes and/or turns on expression of lethal genes. As a result, the strain dies in the nonpermissive environment such as outside the host animal in excreted fecal matter. Such microbes, thus have an environmentally limited viability system. The sequence inserted into the chromosomal *asd* gene site contained promoters, which can be unidirectional or bidirectional, as well as transcription terminators (see for example, WO1996/US/09774, Figures 4, 8, 11, and 13). For bidirectional promoters, it is also desirable to insert transcription terminators on both sides of the inserted promoter sequence, which is operably linked to a coding region, in order to prevent transcription in both directions outside the boundaries of the insert. This prevents transcription on both sides of the promoter.

It can also be desirable in some instances to include a transcription terminator in an insertion cassette containing a reporter gene sequence which is operably linked to a promoter sequence. Such a use of a reporter gene insert into a deleted gene can enable an easy distinction between the vaccine strains and wild-type pathogens. For example, the *xylE* reporter gene can be inserted into a chromosomal mutant gene to evaluate complementation of specific mutational lesions and to verify the existence of both the wild-type allele present on a low copy number plasmid and the mutant allele in the chromosome. Colonies with bacteria that express the *xylE* gene turn yellow upon spraying with catechol solution. However, expression of an *xylE* cassette without a promoter can be variable depending upon how the particular gene into which the *xylE* cassette was inserted is regulated in response to environmental stimuli. Inclusion of a strong promoter in the cassette results in constitutive expression of the *xylE* gene. Because of the strength of the inserted promoter, however, RNA transcription can potentially proceed beyond the insertion. To prevent this, a transcription terminator can be inserted after the C-terminal coding region of the reporter gene. Thus, in this instance also, the insertion of a cassette containing a promoter into a chromosomal gene necessitated the further insertion of a transcription terminator.

It can also sometimes be desirable to insert coding sequences into a deletion mutation that might express regulatory sequences to serve either as activators of gene expression or repressors of gene expression so as to influence properties of other genes within the cell in a desired manner. A transcription terminator can be advantageously incorporated into such insertion sequences to insure that neither the deletion nor the inserted sequence affects expression of adjacent genes.

In other instances, the inclusion of a transcription terminator in an insertion sequence can function to regulate downstream gene expression in an operon. This can provide a unique approach to preventing expression of one or more genes downstream from the insertion and in such instances in which the downstream gene or genes code for gene products involved in biosynthetic, virulence or regulatory gene products, such insertions attenuate the microbe without altering the coding sequence for the downstream gene product.

In one embodiment of the present invention, the bacterial strains are attenuated derivatives of a pathogenic strain. By derivative or derived strain, reference is made to a strain that has been genetically modified from its parent from which it is descended. By pathogenic it is meant that the microbe is capable of causing disease or impairing normal physiological functioning. Reference to attenuation is intended to mean that a particular microbe strain is incapable of inducing a full suite of symptoms of the disease state that is normally associated with its virulent pathogenic counterpart. Thus, attenuation encompasses a state of diminished virulence or ability to produce disease conditions and the attenuated microorganisms are not necessarily completely absent of any ability to impair normal physiological functioning of the host. In addition, an attenuated microbe is not necessarily incapable of ever functioning as a pathogen, but the particular microbe being used is attenuated with respect to the particular individual being treated.

The microbial strains of the present invention are attenuated by virtue of their containing an attenuating insertional mutation comprising a transcription terminator in one or more genes that renders the microorganism attenuated. In one embodiment, the strains have at least two attenuating mutations, at least one of which comprises a transcription terminator. Each of the mutations acts to attenuate the microorganism and, in combination, significantly increases the probability that the microorganism will not revert to wild-type virulence. Attenuating mutations can be in biosynthetic genes, regulatory genes and/or genes involved in virulence. (See Doggett and Brown, in *Mucosal Vaccines,* Kiyono et al., Eds., Academic Press, San Diego, 1996 pp 105-118). Examples of mutations include, but are not limited to a mutation in a *pab* gene, a *pur* gene, an *aro* gene, *asd*, a *dap* gene, *nadA*, *pncB*, *galE*, *pmi*, *fur*, *rpsL, ompR*, *htrA*, *hemA*, *cdt, cya*, *crp, phoP*, *phoQ*, *rfc*, *poxR*, *galU* and combinations thereof. The skilled artisan will readily appreciate that any suitable gene mutation can be used in the present invention so long as the mutation of that gene renders the microorganism attenuated.

The transcription terminator insertion can be accompanied by additional mutagenic changes in the target gene. Thus, a transcription termination insertion can be in a gene in which one or more base pair changes have been introduced, or a gene which has undergone a deletion, frame shift or the like. In a particularly preferred embodiment, the gene has undergone a defined deletion which can be a deletion of the coding region, a deletion of the regulatory region for that gene or a deletion of the entire gene including the regulatory regions. In such instances in which the gene is entirely deleted, the transcription terminator is inserted into the gene site which the gene would other wise occupy.

Defined deletions are specific deletions in genes which can be made using methods known in the art. These methods involve initially selecting a gene in which the deletion is to be generated. In one approach the gene can be selected from a genomic library obtained commercially or constructed using methods well known in the art (Sambrook et al., Molecular Cloning: A Laboratory Manual, 2nd Ed., 1989, Cold Spring Harbor Press, Cold Spring Harbor, N.Y.). Clones containing the gene are isolated from the genomic library by complementation of a strain which contains a mutation in the same gene. Alternatively, when the DNA sequence of the gene is known, selected primers for the polymerase chain reaction method (PCR) can amplify the gene, often with some flanking sequence, from a sample ofbacteria or from purified genomic DNA and the PCR product can be inserted into a cloning vector.

A specific deletion in the selected gene can be generated by either of two general methods. The first method generates a mutation in a gene isolated from a population of clones contained in a genomic DNA library using restriction enzymes and the second method generates the mutation in a gene of known sequence using PCR.

Using the first method, the position of the gene on a vector is identified using transposon tagging and a restriction map of the recombinant DNA in the vector is generated. Information derived from the transposon tagging allows all or a portion of a gene to be excised from the vector using the known restriction enzyme sites.

The second method which is based upon PCR methodology can be used when the DNA sequence of the gene is known. According to this method, divergent PCR primers amplify the upstream and downstream regions flanking a specified segment of DNA to be deleted from the gene and generate a PCR product consisting of the cloning vector and upstream and downstream flanking nucleotide sequences (Innes et al. Eds., PCR Protocols, 1990, Academic Press, New York). In a variation of this method, PCR products are produced representing portions of the gene or flanking sequence, which are then joined together in a cloning vector.

The DNA containing the mutant gene can be introduced into the bacterial host by transformation using chemical means or electroporation, by recombinant phage infection, or by conjugation. In preferred embodiments the mutant gene is introduced into the chromosomes of the bacteria which can be accomplished using any of a number of methods well known in the art such as, for example, methods using temperature-sensitive replicons (Hamilton et al., J. Bacteriol. 171:4617-4622, 1989), linear transformation of *recBC* mutants (Jasin et al., J. Bacteriol. 159:783-786, 1984), or host restricted replicons known as suicide vectors (Miller et al., J. Bacteriol. 170:2575-2583, 1988). The particular method used is coupled with an appropriate counter selection method such as, for example, fusaric acid resistance or sucrose resistance followed by subsequent screening for clones containing the mutant allele based upon phenotypic characteristics or by using PCR, nucleic acid hybridization, or an immunological method.

The attenuated microbe mutants of the present invention can be used in the form of vaccines to deliver recombinant antigens to vertebrate hosts. Thus, it is apparent that the present invention has wide applicability to the development of effective recombinant vaccines against bacterial, fungal, parasite or viral disease agents in which local immunity is important and might be a first line of defense. Some examples are recombinant vaccines for the control of bubonic plague caused by *Yersinia pestis,* of gonorrhea caused by *Neisseria gonorrhea*, of syphilis caused by *Treponema palladium*, and of venereal diseases as well as eye infections caused by *Chlamydia trachomatis*. Species of *Streptococcus* from both group A and group B, such as those species that cause sore throat or heart disease, *Neisseria meningitides, Mycoplasma pneumoniae*, *Haemophilus influenzae*, *Bordetella pertussis, Mycobacterium tuberculosis*, *Mycobacterium leprae, Streptococcus pneumoniae*, *Brucella abortus*, *Vibrio cholerae*, *Shigella species, Legionella pneumophila*, *Borrelia burgdorferi*, *Rickettsia* species, *Pseudomonas aeruginosa*, and pathogenic *E. coli* such as APEC, ETEC, EPEC, UTEC, EHEC, and EIEC strains are additional examples of microbes within the scope of this invention from which genes could be obtained. Recombinant anti-viral vaccines, such as those produced against influenza viruses, are also encompassed by this invention. Recombinant anti-viral vaccines can also be produced against viruses, including RNA viruses such as Picomaviridae, Caliciviridae, Togaviridae, Flaviviridae, Coronaviridae, Rhabdoviridae, Filoviridae, Paramyxoviridae, Orthomyxoviridae, Bunyaviridae, Arenaviridae, Reoviridae or Retroviridae; or DNA viruses such as Hepadnaviridae, Paroviridae, Papovaviridae, Adenoviridae, Herpesviridae or Poxviridae. Recombinant vaccines to protect against infection by pathogenic fungi, protozoa or parasites are also contemplated by this invention.

Thus, in one set of embodiments, the present invention can be described as a vaccine for the immunization of a human comprising a live attenuated derivative of a pathogenic microbe wherein the derivative contains a transcription terminator insertion in a target gene to diminish or abolish expression of that gene and attenuate the microbe as well as to prevent prevent expression of sequences downstream from the target gene. The attenuated microbe is also capable of expressing a recombinant gene derived from an organism that is a pathogen of or that produces an allergen to the human or animal. In embodiments in which the immunogenic component of the vaccine is an allergen of the host, such a vaccine can be used in an exposure regimen designed to specifically desensitize an allergic host. In other embodiments, the recombinant gene expresses a gamete-specific antigen which is capable of eliciting an immune response that confers an antifertility effect upon the immunized individual (See, U.S. Pat. No. 5,656,488).

The attenuated microbes of this invention can additionally'be used as vectors for the synthesis of various host proteins. Because the avirulent microbes of this invention are able to traverse a variety of immunocompetent structures including gut-associated lymphoid tissue (GALT), mesenteric lymph nodes and spleen after introduction into the host, such microbes can be used to target a variety of immunoregulatory products. Accordingly, one or more genes encoding immunoregulatory proteins or peptides can be recombinantly introduced into the attenuated microbes such that when the microbes taking up residence in the appropriate immunocompetent tissue are capable of expressing the recombinant product to suppress, augment or modify the immune response in the host. Examples of immunoregulatory molecules include but are not limited to: colony stimulating factors (macrophage, granulocyte, or mixed), macrophage chemotoxin, macrophage inhibition factor, leukocyte inhibitory factors, lymphotoxins, blastogenic factor, interferon, interleukins, tumor necrotizing factor, cytokines, and lymphokines.

The attenuated microbes of the present invention are also contemplated for use to deliver and produce pharmacologically active products that might stimulate or suppress various physiological functions (i.e., growth rate, blood pressure, etc.). In such microbes, the recombinant gene encodes said pharmacologically active products.

The recombinant gene of the microbes of the present invention can be incorporated into a "balanced-lethal" system which selects for microorganisms containing and capable of expressing the recombinant gene by linking the survival of the microorganism to the continued presence of the recombinant gene. "Balanced-lethal" mutants of this type are characterized by a lack of a functioning native chromosomal gene encoding an enzyme which is essential for cell survival, preferably an enzyme which catalyzes a step in the biosynthesis of diaminopimelic acid (DAP) and even more preferably a gene encoding beta aspartate semialdehyde dehydrogenase (Asd). DAP pathway enzymes and Asd are required for cell wall synthesis. The mutants also contain a first recombinant gene which can serve to complement the non-functioning chromosomal gene and this is structurally linked to a second recombinant gene encoding the desired product. Loss of the complementing recombinant gene causes the cells to die by lysis when the cells are in an environment where DAP is lacking. This strategy is especially useful since DAP is not synthesized by eukaryotes and, therefore, is not present in immunized host tissues. Methods of preparing these types of "balanced lethal" microbes are disclosed in U.S. Pat. No. 5,672,345.

By immunogenic agent is meant an agent used to stimulate the immune system of an individual, so that one or more functions of the immune system are increased and directed towards the immunogenic agent. Immunogenic agents include vaccines. Immunogenic agents can be used in the production of antibodies, both isolated polyclonal antibodies and monoclonal antibodies, using techniques known in the art.

An antigen or immunogen is intended to mean a molecule containing one or more epitopes that can stimulate a host immune system to make a secretory, humoral and/or cellular immune response specific to that antigen.

An epitope can be a site on an antigen to which an antibody specific to that site binds. An epitope could comprise 3 amino acids in a spatial conformation which is unique to the epitope; generally, an epitope consists of at least 5 amino acids and more usually, at least 8-10 amino acids. The term "epitope" is intended to be interchangeable with the term "antigenic determinant" as used herein. The term "epitope" is also intended to include T-helper cell epitopes in which an antigenic determinant is recognized by T-helper cells through association with major histocompatibility complex class II molecules. In addition, the term epitope includes any antigen, epitope or antigenic determinant which is recognized by cytotoxic T cells when presented by a MHC class I molecule on the surface of an antigen presenting cell. A cytotoxic T cell epitope can comprise an amino acid sequence of between about 6 to about 11 amino acids, and preferably comprises a sequence of 8 or 9 amino acids.

By vaccine is meant an agent used to stimulate the immune system of an individual so that protection is provided against an antigen not recognized as a self-antigen by the immune system. Immunization refers to the process of inducing a continuing high level of antibody and/or cellular immune response in which T-lymphocytes can either kill the pathogen and/or activate other cells (e.g., phagocytes) to do so in an individual, which is directed against a pathogen or antigen to which the organism has been previously exposed. Although the phrase "immune system" can encompass responses of unicellular organisms to the presence of foreign bodies, in this application the phrase is intended to refer to the anatomical features and mechanisms by which an individual produces antibodies against an antigenic material which invades the cells of the individual or the extra-cellular fluid of the individual and is also intended to include cellular immune responses. In the case of antibody production, the antibody so produced can belong to any of the immunological classes, such as immunoglobulins, A, D, E, G or M. Of particular interest are vaccines which stimulate production of immunoglobulin A (IgA) since this is the principle immunoglobulin produced by the secretory system of warm-blooded animals, although vaccines of the invention are not limited to those which stimulate IgA production. For example, vaccines of the nature described herein are likely to produce a broad range of other immune responses in addition to IgA formation, for example cellular and humoral immunity. Immune responses to antigens are well studied and widely reported. A survey of immunology is provided in Elgert, Klaus D., Immunology, Wiley Liss, Inc., (1996); Stites et al., Basic & Clinical Immunology; 7th Ed., Appleton & Lange, (1991) the entirety of which are incorporated herein by reference.

An "individual" receiving a vaccine or carrier microbe of this invention, is intended to include vertebrate such as fishes, amphibians, reptiles, birds, and mammals. All vertebrates have a functional immune system and respond to antigens by producing antibodies so that, all vertebrates are capable of responding to vaccines. Particularly preferred individuals are mammals, such as humans, dogs, cats, pigs cows and the like. Fishes and birds are also commonly raised commercially and the compositions and methods of the present invention can also advantageously administered to fishes and birds.

Microbes as used herein can include bacteria, protozoa and unicellular fungi. The term parasite as used herein is intended to encompass both endoparasites and ectoparasites including protozoans such as species of Plasmodium and Toxoplasma, species of Entamoeba, Leishmania and Trypanosoma and helminths such as trematodes, cestodes and nematodes as well as mites ticks and fleas. Viruses as used herein can include RNA viruses such as Picornaviridae, Caliciviridae, Togaviridae, Flaviviridae, Coronaviridae, Rhabdoviridae,Filoviridae, Paramyxoviridae,Orthomyxoviridae, Bunyaviridae, Arenaviridae, Reoviridae and Retroviridae; and DNA viruses such as Hepadnaviridae, Paroviridae, Papovaviridae, Adenoviridae, Herpesviridae and Poxviridae.

Reference to a recombinant gene is intended to mean genetic material that is transferred from a first organism into a second organism which upon reproduction gives rise to descendants containing the same genetic material. Generally, such exchange of genetic material from the first organism to the second organism does not normally take place in nature. Recombinant gene as used herein is intended to reference genetic material incorporated into a microbe by human intervention.

The term gene as used herein in its broadest sense represents any biological unit of heredity. It is not, however, necessary that the recombinant gene be a complete gene as is present in the parent organism and capable of producing or regulating the production of a macromolecule such as for example, a functioning polypeptide. The recombinant gene may, thus, encode all or part of an antigenic product. Furthermore, the recombinant gene can also include DNA sequences that serve as promoters, enhancers or terminators and DNA sequences that encode repressors or activators that regulate expression of a recombinant gene encoding all or part of an antigen. A recombinant gene can also refer to gene fusions which encode polypeptide fusion products. The encoded gene product can, thus, be one that was not found in that exact form in the parent organism. For example, a functional gene coding for a polypeptide antigen comprising 100 amino acid residues can be transferred in part into a carrier microbe so that a peptide comprising only 75, or even 10, amino acid residues is produced by the cellular mechanisms of the host cell. However, if this gene product can serve as an antigen to cause formation of antibodies against a similar antigen present in the parent organism or as a T-cell epitope recognized by T-helper cells, the gene is considered to be within the scope of the term gene as defined in the present invention. Alternatively, if the amino acid sequence of a particular antigen or fragment thereof is known, it is possible to chemically synthesize the DNA fragment or analog thereof by means of automated gene synthesizers or the like and introduce said DNA sequence into the appropriate expression vector. This might be desirable in order to use codons that are preferred codons for high level expression in Salmonella. At the other end of the spectrum is a long section of DNA coding for several gene products, one or all of which can be antigenic. For example, such a long section of DNA could encode 5 to 15 proteins necessary for the synthesis of fimbrial antigens (fimbriae), which mediate adhesion of pathogens to host cells (Baumler et al., supra). The induction of an immune response against fimbriae can provide protection against the pathogen. Thus, a gene as defined and claimed herein is any unit of heredity capable of producing an antigen. The gene can be of chromosomal, plasmid, or viral origin. It is to be understood that the term gene as used herein further includes DNA molecules lacking introns such as, for example, is the case for cDNA molecules, so long as the DNA sequence encodes the desired gene product.

In order for the gene to be effective in eliciting an immune response, the gene must be expressed. Expression of a gene means that the information inherent in the structure of the gene (the sequence of DNA bases) is transformed into a physical product in the form of an RNA molecule, polypeptide or other biological molecule by the biochemical mechanisms of the cell in which the gene is located. The biological molecule so produced is referenced as the gene product. The term gene product as used here refers to any biological product or products produced as a result of the biochemical reactions that occur under the control of a gene. The gene product can be, for example, an RNA molecule, a peptide, or a product produced under the control of an enzyme or other molecule that is the initial product of the gene, i.e., a metabolic product. For example, a gene can first control the synthesis of an RNA molecule which is translated by the action of ribosomes into an enzyme which controls the formation of glycans in the environment external to the original cell in which the gene was found. The RNA molecule, the enzyme, and the glycan are all gene products as the term is used here. Any of these as well as many other types of gene products, such as glycoproteins, glycolipids and polysaccharides, will act as antigens if introduced into the immune system of an individual. Protein gene products, including glycoproteins and lipoproteins, are preferred gene products for use as antigens in vaccines.

In order for a vaccine to be effective in stimulating cellular immunity or in producing antibodies, the antigenic materials must be released and/or presented in such a way to trigger the induction of cellular immunity and/or induce the antibody-producing mechanism of the vaccinated individual. Therefore, the microbe carrier of the gene product must be introduced into the individual. In order to stimulate a preferred response of the gut-associated lymphoid tissue (GALT) or bronchus-associated lymphoid tissue (BALT), introduction of the microbe or gene product directly into the gut or bronchus is preferred, such as by oral administration, gastric intubation or intranasally in the form of aerosols, although other methods of administering the vaccine, such as intravenous, intraperitoneal, intramuscular, subcutaneous injection or intramammary or intrapenial or vaginal or rectal administration, are possible.

The avirulent microbe can be used as a carrier microbe, for example, for an antigen, and once the carrier microbe is present in the individual, the antigen needs to become available to the individual's immune system. This can be accomplished when the carrier microbe dies so that the antigen molecules are released. Of course, the use of "leaky" avirulent mutants that release the contents of the periplasm without lysis is also possible. Alternatively, a gene can be selected that controls the production of an antigen that will be made available by the carrier cell to the outside environment prior to the death of the cell. In this way, it is possible to use a viable microbe that will persist in the vaccinated individual, for example in its Peyer's patches or other GALT, and continue to produce antigen, thereby continually inducing antibody formation and/or a cellular immune response. A preferred gene product under these circumstances is a product that is transferred through the cell membrane of the avirulent carrier microbe into the external environment or a product that becomes attached to or embedded in the external membrane so that all or part of the gene product is exposed to the environment. Typical of this latter type of gene product are antigens normally found on the surface of the organism against which protection is desired. If these antigens are transported to the bacterial cell surface in a normal manner, antibody formation against the antigens will be enhanced.

The use of pathogens to deliver antigens from other pathogens to the GALT or BALT would be inappropriate if it were not for the fact that such pathogens can be rendered avirulent while retaining ability to colonize these tissues.

The organism from which the recombinant gene is derived can be any pathogen or may be an organism that produces an allergen or other antigen to which a human or an animal such as a dog or cat, can be sensitive. Allergens are substances that cause an allergic reaction for which the individual can be vaccinated. Many different materials can be allergens, such as animal dander and pollen, and the allergic reaction of individuals will vary for any particular allergen. It is possible to induce tolerance to an allergen in an individual that normally shows an allergic response. The methods of inducing tolerance are well-known and generally comprise administering the allergen to the individual in increasing dosages. Further discussion of tolerance induction is given in the Barrett textbook previously cited. Lastly, the host individual itself can serve as a source of genetic material when immunoregulatory genes or genes for other pharmacologically active substances are being expressed by the vectors.

Administration of a live vaccine of the type disclosed above to an individual can be by any known or standard technique. These include oral ingestion, gastric intubation, or broncho-nasal-ocular spraying. All of these methods allow the live vaccine to easily reach the GALT or BALT cells and induce antibody formation and cell mediated immunity and are the preferred methods of administration. Other methods of administration, such as intravenous injection, that allow the carrier microbe to reach the individual's blood stream can be acceptable. Intravenous, intramuscular or intramammary injection are also acceptable with other embodiments of the invention, as is described later.

Any of a number of commonly used recombinant DNA techniques can be used in producing the attenuated microbes of the present invention which are capable of expressing a recombinant gene. Following ligation to a plasmid, phage or cosmid vector the recombinant molecules so formed can be transferred into a host cell by various means such as conjugation, or transformation (uptake of naked DNA from the external environment, which can be artificially induced by the presence of various chemical agents, such as calcium ions), including electroporation. Other methods such as transduction are also suitable, wherein the recombinant DNA is packaged within a phage such as transducing phage or cosmid vectors. Once the recombinant DNA is in the carrier cell, it may continue to exist as a separate autonomous replicon or it may insert into the host cell chromosome and be reproduced along with the chromosome during cell division.

Once the genetic material has been transferred, the microbes containing the transferred genetic material are selected.

The immunization dosages required will vary with the antigenicity of the gene product and need only be an amount sufficient to induce an immune response. Routine experimentation will easily establish the required amount. Multiple dosages are used as needed to provide the desired level of protection.

The pharmaceutical carrier or excipient in which the vaccine is suspended or dissolved may be any solvent or solid or encapsulating material such as for a lypholized form of the vaccine. The carrier is non-toxic to the inoculated individual and compatible with the microorganism or antigenic gene product. Suitable pharmaceutical carriers are known in the art and, for example, include liquid carriers, such as normal saline and other non-toxic salts at or near physiological concentrations, and solid carriers, such as talc or sucrose. Gelatin capsules can serve as carriers for lypholized vaccines. Adjuvants may be added to enhance the antigenicity if desired. When used for administering via the bronchial tubes, the vaccine is preferably presented in the form of an aerosol. Suitable pharmaceutical carriers and adjuvants and the preparation of dosage forms are described in, for example, Remington's Pharmaceutical Sciences, 17th Edition, (Gennaro, Ed., Mack Publishing Co., Easton, Pa., 1985).

Immunization of an individual with a pathogen-derived gene product can also be used in conjunction with prior immunization with the avirulent derivative of a pathogenic microorganism acting as a carrier to express the gene product specified by a recombinant gene from a pathogen. Such parenteral immunization can serve as a booster to enhance expression of the secretory immune response once the secretory immune system to that pathogen-derived gene product has been primed by immunization with the carrier microbe expressing the pathogen-derived gene product to stimulate the lymphoid cells of the GALT or BALT. The enhanced response is known as a secondary, booster, or anamnestic response and results in prolonged immune protection of the host. Booster immunizations may be repeated numerous times with beneficial results.

### Industrial Application

The microbes of the present invention contain attenuating mutations comprising an insertion of at least one transcription terminator into a chromosomal gene. The attenuated microbes can be used for vaccines and/or for delivery vehicles for a desired gene product. The microbes can thus serve as live attenuated vaccines capable of eliciting an immunogenic response and a protective immunity in the individual to which it is administered. Vaccines strains can also be constructed to contain and express a recombinant gene from a pathogen to immunize an individual against the pathogen or an allergen for use in desensitizing an individual. The attenuated microbes of this invention can additionally be used as vectors for the synthesis of various host proteins such as immunoregulatory substances or for the deliver and production of pharmacologically active products that might stimulate or suppress various physiological functions (i.e., growth rate, blood pressure, etc.). Moreover, the attenuated microbes of the present invention can be used for the commercial production of recombinant proteins.

Preferred embodiments of the invention are described in the following examples. Other embodiments within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims which follow the examples.

Strains and plasmids used in the studies reported herein are listed in Tables 1A and 1B.

**TABLE 1**

| A. Bacterial Strains | | | |
|---|---|---|---|
| **Strain** | **Description** | **Genotype** | **Source/ Derivative** |
| χ3339 | *S*. *typhimurium* SL1344 wild-type, isolated from liver of moribund mouse after peroral inoculation. | *hisG46* Su^{r} Sm^{r} | Curtiss Collection |
| χ3761 | *S. typhimurium* UK-1 wild-type strain obtained as a chicken passaged spleen isolate. | wild type | Curtiss Collection |
| χ8087 | Defined deletion derivative of *S. typhimurium* UK-1 containing *ΔphoP22*. | *ΔphoP22* | χ3761 |
| χ8088 | Defined deletion derivative of *S*. *typhimurium* UK-1 containing *ΔphoQ1*. | *ΔphoQ1* | χ3761 |
| χ8089 | Defined deletion derivative of *S. typhimurium* UK-1 containing *ΔphoPQ23*. | *ΔphoPQ23*. | χ3761 |
| χ8297 | Defined deletion derivative of *S. typhimurium* SL1344 containing *ΔphoP23*. | *ΔphoPQ23* *hisG46* | χ3339 |
| χ8299 | Defined deletion derivative of *S. typhimurium* SL1344 containing Δ*phoP22*. | *ΔphoP22* *hisG46* | χ3339 |
| χ8429 | Defined deletion derivative of *S. typhimurium* UK-1 containing *ΔphoP24*. | *ΔphoP24* | χ3761 |
| χ8430 | Defined deletion derivative of *S*. *typhimurium* SL1344 containing *ΔphoQ1*. | *ΔphoQ1* *hisG46* | χ3339 |
| χ8431 | Defined deletion derivative of *S. typhimuriumUK-1* containing *ΔphoP1918*. | *ΔphoP1918* | χ3761 |
| χ8432 | Defined deletion derivative of *S*. *typhimurium* SL1344 containing *ΔphoP1918*. | Δ*phoP1918* *hisG46* | χ3339 |
| χ8597 | Defined deletion derivative of *S*. *typhimurium* SL1344 containing *ΔphoP24*. | *ΔphoP24* *hisG46* | χ3339 |
| χ3385 | Cured χ3384 of the 100 kb plasmid. | *hsdL6 galE496 trpB2 his-6166 rpsL xyl-404 metE551 metA22 lamB*⁺ *(E. coli) Δzja::*Tn*10 hsdSA29 val* | χ3000 |
| χ4669 | Received from Eduardo Groisman as 7953s. | *phoP::*Tn*10* | 14028s |
| χ2981 | Derivative of *E. coli* K-12 containing *ΔasdA4.* | *Δ(proB-lacYZ)* λ⁻ *T3*^{*r*}*-ΔasdA4 zhf-2::*Tn*10 cycA1* | χ354 |
| MGN-023 | An Asd⁻ Tet^{s} derivative of *S*. *typhimurium* UK-1 χ3761 obtained after counter selection on fusaric acid of the pMEG-006 integrant. | *ΔasdA16* | χ3761 |
| MGN-069 | Obtained by transducing MGN-064 with P22HT*int* grown on χ4669 then plating transductants on fusaric acid agar. This is one of the FA^{R} Tc^{S} derivative of MGN-064 which was confirmed as Pho⁻, Tc^{s}, while still allowing replication of *pir* replicons. The strain is intended for use as an intermediate for screening phoP from *pir* dependent plasmid libraries. | *ΔphoP13* | χ3385 MGN-064 |
| MGN-219 | Defined deletion intermediate obtained by electroporating χ3761 with large amounts of pMEG-082. | | χ3761 |
| MGN-220 | Defined phoP deletion derivative of MGN-219 obtained by selecting for fusaric acid resistant isolate and screening for Pho- phenotype with soft agar overlay method of Kier. | *ΔphoP22* | χ3761 MGN-219 |
| MGN-392 | *S*. *typhimurium* UK-1 containment host obtained from MGN-377 by selecting for fusaric acid resistant, tetracycline sensitive, Asd⁻, excision of pMEG-096 leaving the *PR c2* clone in the *asd* gene defined deletion. Note complementation to wild type virulence not obtained with Asd+ vector pYA810. | *Δasd-17* *::cI857PRc2* | χ3761 MGN-377 |
| MGN-491 | *S. typhimurium* UK-1 containment host MGN-391 transduced to Asd+ using P22HT *int* grown on χ3000. This results in restoration of the wild-type phenotype and normal LD50. Virulence previously observed lacking in MGN-391 when complemented with an Asd⁺ plasmid, pYA810. | wild-type prototroph | χ3761 MGN-377 MGN-391 |
| MGN-617 | *E*. *coli* K-12 *ΔasdA4* suicide donor strain derived from SM10 *λpir*, following transduction with P1 from χ2981 to Tet resistance and Asd-. Fusaric acid resistant isolate selected and confirmed to be Ap^{s}, Tet^{s}, and Asd⁻. | *thi-1 thr-1 leuB6 supE44 tonA21 lacY1 recA RP4-2- Tc:: Mu lpir*, *ΔasdA4 Δzhf- 2::Tn10* | SM10 λpir MGN-595 MGN-614 |
| MGN-740 | The 4.0 kb *XbaI*-*XhoI* of pMEG-201 carrying *ΔphoQ::xylE* cloned into the *Xba*I-*Sal*I sites of pKNG101. Suicide delivery strain for making *ΔphoQ::xylE* defined deletion. Note the orientation of the *xylE* insertion is not confirmed. | *thi-1 thr-1 leuB6 supE44 tonA21 lacY1 recA RP4-2- Tc: : Mu λpir*, *ΔasdA4 Δzhf*-*2::Tn10* | SM10 λpir MGN-595 MGN-614 MGN-617 |
| MGN-795 | An Asd⁻ Tet^{s} derivative of *S. typhimurium* UK-1 containing *ΔasdA19*. | *ΔasdA19* | χ3761 MGN-793 |
| MGN-798 | MGN-795 electroporated with the containment vector pMEG-209, this has *asd* regulated by lambda CI857 (which is present on the plasmid) and the P22 lysis genes regulated by C2 (which is regulated by arabinose). The arabinose regulated P22 C2 repressor in the chromosome of this strain results in no lysis genes expressed when grown in arabinose but death by lysis if grown without arabinose. | *Δasd-19* (is *ΔasdA16*::PB AD.*C2*) | χ3761 MGN-793 MGN-795 |
| MGN-1361 | The *ΔphoP24* terminator intermediate obtained by conjugating MGN-1358, containing pMEG-368 suicide plasmid, with χ3339 and selecting for Ap resistant isolates. This is integrant #4 and has the Pho⁺ phenotype of parent. | *phoP*⁺ ::(pMEG-368Δ*phoP24*) *hisG 46 rpsL xyl* | χ3339 |
| MGN-3635 | Defined deletion derivative of *S. typhimurium* SL1344 containing *ΔphoP24*. | *ΔphoP24* | χ3339 MGN-1361 |
| MGN-2052 | MGN-026 carrying pMEG-549, constructed using inverse PCR to delete the *phoP* (-10 -35 ATG ----TAA sequences) of pEG5381 from E. Groisman | *endA1 hsdR17* (*rk*-,*mk*+) *supE44 thi-1 recA1 gyrA relA1 Δ(lacZYA- argF) U169 λpir deoR (φ80dlacΔ(lacZ) M15)* | DH5α MGN-026 |
| MGN-2083 | Constructed via conjugation of χ3761 with MGN-2054e to integrate the suicide plasmid pMEG-550 *containing ΔphoP1918*. Deletion constructed using inverse PCR on pEG5813 from E.Groisman (*phoPQ*) with deletion of -35 -10 upstream region and ATG to TAA of *phoP*. Isolate #1, Smooth LPS, Deletion confirmed by PCR | *ΔphoP1918* | χ3761 |
| MGN-2084 | Defined deletion derivative of *S. typhimurium* UK-1 containing *ΔphoP1918*. | *ΔphoP1918* | χ3761 |
| MGN-2085 | Defined deletion derivative of *S*. *typhimurium* SL1344 containing *ΔphoP1918*. | *ΔphoP1918* *hisG46* | χ3339 |
| MGN-2086 | Constructed via conjugation of χ3339 with MGN-2054e to integrate the suicide plasmid pMEG-550 *containing ΔphoP1918*. Deletion constructed using inverse PCR on pEG5813 from E.Groisman (*phoPQ*) with deletion of -35 -10 upstream region and ATG to TAA of *phoP*. Isolate #8, Smooth LPS, Deletion confirmed by PCR | *hisG46* *ΔphoP1918* Su^{r} Sm^{r} | χ3339 |

**TABLE I**

| B. Plasmids | | |
|---|---|---|
| **Plasmids** | **Description** | **Source/ Reference** |
| pMEG-076 | The promoter left of lambda PCR amplified using primers PL.5-23 and PL.160-136, from χ2869 and cloned as a *Bam*HI cut Taq ext. polymerase produced 163 bp fragment into the SmaI-BamHI site of pKK232-8, selecting for chloramphenicol resistance. | MEGAN Health, Inc. |
| pMEG-068 | A *phoP* deletion of pMEG-063 retaining *phoQ*. Obtained by digesting pMEG-063 with *Eco*RV removing the 514 bp *Eco*RV fragment within the *phoP* gene and religating to regenerate an *Eco*RV site. No longer complements PhoP⁻ phenotype of MGN-069. | MEGAN Health, Inc. |
| pMEG-086 | An Asd⁺ clone obtained by inserting the 314-1421 bp *asd* PCR product lacking the promoter and SD regions (obtained using primers Asd.314-336 {*Bgl*II} and Asdend 1421-1403 {*Xba*I} with Vent), as a 1115 bp *Bgl*II -blunt fragment, into the *Bam*HI-*Pvu*II sites of pMEG-076. This places *asd* under the regulation of lambda's promoter left. | MEGAN Health, Inc. |
| pMEG-096 | pMEG-096 is the ∼2 kb ClaI fragment of pMEG-088 treated with T4 DNA polymerase and ligated into the Bg1II site of pMEG-006 following treatment with T4 DNA polymerase and CLAP. This provides the lambda PR driven P22 *C2* gene and lambda *cI857* between either part of the *S. typhimurium asd* deletion allowing insertion into the chromosome, providing temperature sensitive regulation of C2 repressor. | MEGAN Health, Inc. |
| pMEG-100 | pMEG-100, an Asd.lysis expression vector intermediate obtained by ligating the 2.26 kb *Bam*HI-*Bgl*II fragment of pMEG-098 containing the asd.lys cartridge into the *Bgl*II site of pMBG-090. | MEGAN Health, Inc. |
| pMEG-105 | Asd.lysis p15A expression vector obtained by deleting the 1.36 kb *Sal*I Km fragment of pMEG-100. Requires host with C2 repressor. | MEGAN Health, Inc. |
| pMEG-106 | The *phoQ* gene was deleted from pMEG-063 by partial digest with *Nde*I and complete digest with *Not*I followed by treatment with T4 DNA polymerase and ligation of these blunt ends, this is isolate #2, which complements Pho phenotype of MGN-220. | MEGAN Health, Inc. |
| pMEG-149 | A *pir* dependent suicide vector which provides a light blue/white screen for inserts in MGN-026 and is mobilized by *incP* containing strains such as CC118 or SM10 lambda *pir*. | MEGAN Health, Inc. |
| pMEG-193 | *Bgl*II partial digest of pYA292, filled in using Klenow and religated | MEGAN Health, Inc. |
| pMEG-195 | p15A expression vector with *Bgl*II sites removed by *Bgl*II partial digest of pMEG-193, filled in using Klenow and religating | MEGAN Health, Inc. |
| pMEG-197 | p15A expression vector intermediate lacking the *trc* promoter, obtained by deleting the *Bam*HI-*Bgl*II fragment from pMEG-195 and religating to remove the *Ptrc* promoter | MEGAN Health, Inc. |
| pMEG-198 | Purified the 2.4 kb (*Kpn*I-Klenowed followed by *Xba*I digest) fragment containing *phoPQ* genes from pMEG-063. Cloned into the *Dra*I-*Xba*I sites of pMEG-197. | MEGAN Health, Inc. |
| pMEG-201 | Cloned the 2.4 kb *Eco*RI-*Hin*dIII fragment of pKNG37 containing *xylE* gene into the *Dra*I-*Nae*I sites of pMEG-198. Both fragments were made blunt-ended. This removed ∼900 bp from *phoQ* coding region. The *xylE* gene is under the control of T4 gene32 promoter and terminator sequences. | MEGAN Health, Inc. |
| pMEG-202 | *Dra*I-*Nae*I defined deletion of pMEG-198, Klenow treatment and religated. This removed ∼900 bp from *phoQ* coding region. | MEGAN Health, Inc. |
| pMEG-205 | The 4.0 kb *Xba*I-*Xho*I of pMEG-201 carrying *ΔphoQ::xylE* cloned into the *Xba*I-*Sa*lI sites of pKNG101. Suicide delivery strain for making *ΔphoQ::xylE* defined deletion. Note the orientation of the *xylE* insertion is not confirmed. | MEGAN Health, Inc. |
| pMEG-210 | A *phoPQ* deletion of pMEG-068, obtained by digesting pMEG-068 with *Eco*RV and *Tth*III1 removing the 1103 bp *Eco*RV-*Tth*III1 fragment encoding the C terminal of *phoP* and the His region of *phoQ*. The linearized plasmid was then treated with T4 DNA polymerase and religated. | MEGAN Health, Inc. |
| pMEG-212 | This clone is the *Bgl*II-*Xba*I fragment of pMEG-210 containing the *phoPQ23* defined deletion ligated into the *Bam*HI-*Xba*I sites of pMEG-149. This is the suicide vector used for constructing *ΔphoPQ23* defined deletion strains. | MEGAN Health, Inc. |
| pMEG-359 | *ΔphoP24* intermediate clone, obtained by digesting the inverse PCR clone pMEG-355, with *Bgl*II and *Eco*RI then ligating to the *trpA* terminator linkers. This clone no longer has a functional pho+ phenotype on acid phosphatase soft agar overlay. | MEGAN Health, Inc. |
| pMEG-368 | A suicide vector for *ΔphoP24* containing the 3.1 kb *EcoR*V fragment of pMEG-359 cloned into the *Sma*I site of pMEG149. This *phoP24* deletion contains the *trpA* terminator with a new *Not*I site but does not modify the sequence of *phoQ*. | MEGAN Health, Inc. |
| pMEG-375 | A chloramphenical and ampicillin resistant suicide vector derived from pMEG-149 by inserting the ∼1.6 kb *Hinc*II-*Xmn*I fragment of pYA800 containing the Cat gene of pACYC184 into the *Ssp*I site of pMEG-149. | MEGAN Health, Inc. |
| pMEG-443 | A suicide vector for *ΔasdA16* obtained by cloning ∼1.5 kb *Hinc*II to *Sph*I fragment of pMEG-006 containing *ΔasdA16* and R6K ori into the *SmaI-Sph*I site of suicide vector pMEG-375. | MEGAN Health, Inc. |
| pMEG-549 | Inverse PCR using pEG-5381; primers were designed to delete *phoP* upstream sequences (-10 and -35) and ATG through TAA to produce *ΔphoP1918*. | MEGAN Health, Inc. |
| pMEG-550 | The *Eco*Rv insert (smaller of the two) fragment from pMEG-549 containing *ΔphoP1918* was ligated into the *Pme*I site of the suicide vector pMEG-375. | MEGAN Health, Inc. |
| pMEG-611 | A chloramphenicol and ampicillin resistant suicided vector derived from pMEG-375 by inserting ∼4.6 kb *Acc*I *-Sph*I fragment of pMEG-221 containing the P22 C2 repressor under the control of *AraC*PBAD in the *ΔasdA16* deletion into the *Sph*I-*Pme*I sites of pMEG-375. | MEGAN Health, Inc. |
| pMEG-685 | T4-*xylE*-omega casstte as cloned into a pBR based replicon. | MEGAN Health, Inc. |
| pYA292 | A p15A replicon containing the *asd* gene of *Salmonella typhimurium*. Plasmid contains the *lacZ* gene. | Galan J.E., K. Nakayama and R. Curtiss III. 1990. Gene 94:29-35. |
| PYA810 | A p15A replicon containing the *asd* gene of *Salmonella typhimurium*. | Washington Univ. |
| pEG5381 | *phoPQ* clone from *S. typhimurium*. | Groisman et al. (1989) PNAS 86: 7077-7081 |
| pWKS30 | A low copy number cloning vector containing a pSC101 origin of replication. | Wang, R. F. and Kushner, S. R. (1991) - |

### EXAMPLE 1

This example illustrates the generation of defined mutants and the insertion of a cassette containing a transcription terminators into the deleted gene site. These studies show the importance of incorporating a transcription terminator into an inserted sequence to avoid effects of the insertion on adjacent genes.

### Generating a defined deletion in the asd gene

The approach of using defined deletions was prompted by our observation in complementation studies that transposon generated mutation can sometimes extend into genes beyond the target gene and influence properties of importance for immunogenicity.

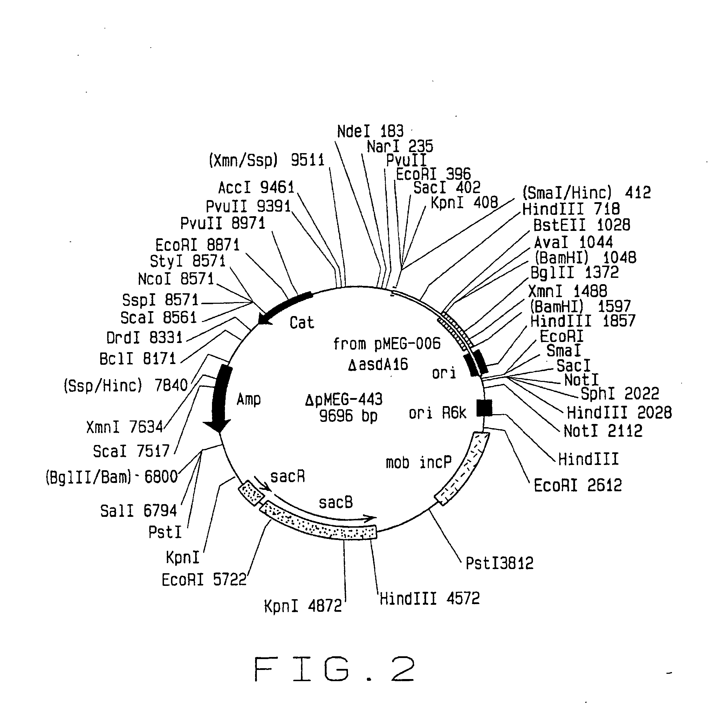

In order to generate defined deletions in the *asd* gene, the *S*. *typhimurium asd* gene was cloned and sequenced. The 1735 base pair sequence thus obtained contains 313 base pairs of 5' upstream sequence, a coding sequence of 1104 base pairs specifying a 368 amino acid protein followed by a three base pair stop codon and 315 base pairs of 3' downstream sequence. Using inverse PCR to delete base pairs 287 to 1421 of the sequence with insertion of a *Bgl*II site in the center, a suicide vector pMEG-443 (Fig. 2) to use in allele replacement for the *ΔasdA16* mutation was constructed. This suicide vector can be introduced into attenuated *Salmonella* vaccine strains either by electroporation or by conjugational transfer from the donor strain MGN-617, which possesses an integrated *incP* replicon which promotes conjugational transfer of the suicide vector. MGN-617 also has a lambda prophage integrated into the chromosome, specifying the *pir* function which is necessary for the vegetative replication of plasmids that have the R6K origin of replication. When pMEG-443 is transferred conjugationally or by electroporation into a *Salmonella* strain, the plasmid fails to replicate due to the absence of the *pir* gene product. However, by selecting for simultaneous resistance to ampicillin (Amp^{r}) and choramphenicol Cam^{r}) whose resistances are encoded by the *bla* (*amp*) and *cat* genes on pMEG-443, a single crossover event using sequence homology flanking the deleted *asd* gene enables the suicide vector to associate with and recombine into the bacterial chromosome. Individual Amp^{r} Cam^{r} colonies can be picked and purified and small cultures grown up to then plate on agar medium containing 5% sucrose but lacking ampicillin and chloramphenicol and importantly containing DAP. The *sacB* gene on the suicide vector encodes a levansucrase which hydrolyzes sucrose leading to toxicity and bacterial cell death under conditions of low osmolarity. Thus if a second crossover event occurs to excise the suicide vector from the chromosome, bacterial cells can survive due to loss of the plasmid vector. In some cases, the crossover event occurs on the other side of the *asd* deletion from the site of the first crossover event to lead to allele replacement so that the defined deletion mutation *ΔasdA16* replaces the wild-type *asd* gene in the chromosome. This procedure can be used with a great diversity of *Salmonella* strains of differing serotypes to introduce the *ΔasdA16* mutation with its 1135 base pair deletion eliminating the entire coding sequence for the structural *asd* gene and replacing it with a *Bgl*II recognition sequence. Such a selection resulted in the *S. typhimurium* UK-1 strain MGN-023 derived from its χ3761 parent. When many of the Asd⁺ plasmids were introduced into MGN-023, the Asd⁺ recombinants displayed the same growth rate, the same ability to colonize murine intestinal tract and internal lymphoid tissues, and to exhibit pathogenicity as revealed by LD₅₀ determination, as did the wild-type Asd⁺ parent χ3761.

### Use of transcription terminators in insertions into deleted chromosomal asd gene site

In designing a strategy for regulating the survivability of microbes, we constructed *asd* deletion mutants with a temperature-sensitive insertion cassette containing genes which regulate genes governing survival of the microbe. The genes governing survival were on a plasmid which also contained a complementing *asd* gene. However, in order to limit the effects of the insertion cassette, transcription terminators were also incorporated into the cassettes.

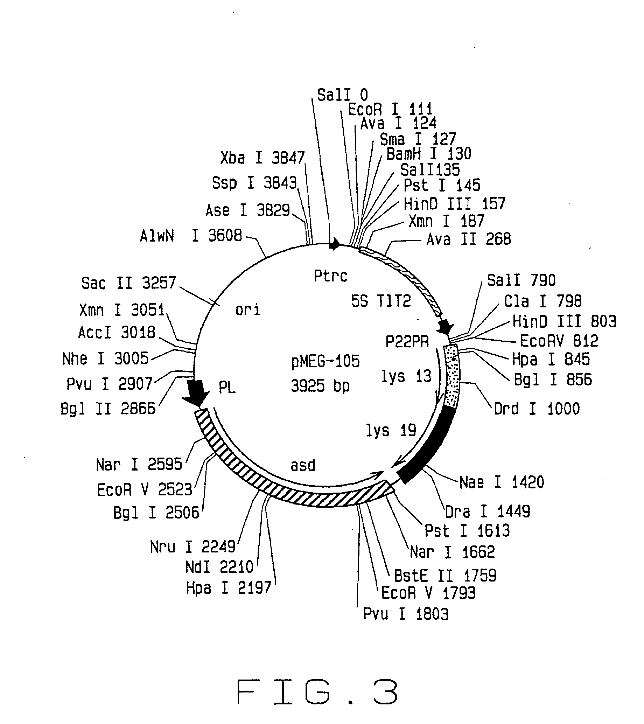

The *asd* gene in containment vector pMEG-105 (Fig. 3) complements a deleted chromosomal gene. Expression of the plasmid *asd* gene makes use of the λ promoter left (P_{L}) which functions only when there is no λ cI repressor protein present in the cytoplasm. The vector was constructed to also contain P22 phage lysis genes *lys-13* and *lys-19* which are expressed under the control of the P22 promoter right (P_{R}). To preclude the expression of these lethal genes it was necessary that the bacterial cell produce copious quantities of the P22 *c2* gene product which is the repressor for P_{R}. We constructed *ΔasdA17* mutants with an insertion cassette in the deleted *asd* gene in which the insertion cassette contained and expressed either λ cI repressor protein or *cI857* gene product. The λ cI repressor blocked expression of the plasmid encoded *asd* gene. This blocking action was temperature-sensitive so that the product would shut off *asd* gene expression on the pMEG-105 plasmid at ambient temperatures encountered in the environment, but permit *asd* gene expression at the body temperatures of an immunized animal host under conditions that would thermally inactivate the *cI857* gene product. The *ΔasdA17* deletion-insertion construct also caused expression of the P22 *c2* gene under conditions in the animal body in which the *cI857* gene product was thermally inactivated so that the P22 C2 protein would be synthesized to repress expression of the pMEG-105 encoded *lys-13* and *lys-19* genes due to the repression of the P22 P_{R} by the C2 repressor. The *cI857* λP_{R}P22*c2* construct was also constructed to include the *rrnB*T1T2 transcription terminators (Fig. 1) at the C-terminal end of the *cI857* gene to preclude transcription through the end of the *asd* deletion. This fragment was inserted into and in place of the deletion in the *asd* gene and placed in a suicide vector to yield pMEG-096 (Fig. 4). pMEG-096 can be introduced either by electroporation or conjugation into the suicide vector donor MGN-617. Since pMEG-096 possesses the tetracycline resistance determinant, the first recombination event for insertion of the suicide vector into the chromosome of a recipient *Salmonella* strain can be achieved by selecting for tetracycline resistance. Tetracycline resistance, as stated above, specifies sensitivity to fusaric acid and one can then select for a second crossover event by selecting fusaric acid resistant isolates.

Strain MGN-392 was constructed by replacing the wild-type *asd*⁺ gene with the *ΔasdA17*::TT*c1857*λP_{R}P22*c2* construction where TT is the transcription terminator, *rrnB*T1T2 (Fig. 1). This strain has an obligate requirement for DAP and expresses the C2 repressor protein when grown at high temperature due to thermal inactivation of the temperature-sensitive CI857 repressor, but fails to express the *c2* gene when grown at low temperature under conditions in which the *cI857* gene product is a functional repressor binding to λ P_{R} to preclude transcription of the P22 *c2* gene.

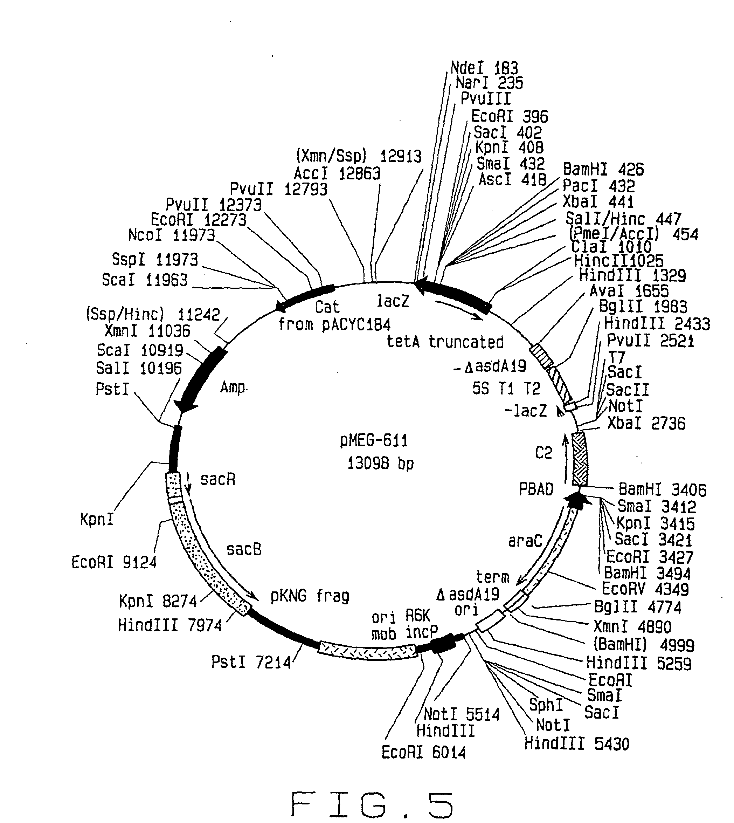

Another construction by insertion of the P22 *c2* gene within the *Δasd* deletion mutation was engineered so that *c2* gene expression would be controlled by an *araC*P_{BAD} activator/repressor system dependent on the presence or absence of arabinose. By growing a strain with an *araC*P_{BAD}*c2* construction in the chromosome in the presence of arabinose, copious quantities of C2 protein would accumulate and serve to repress lethal genes such as *lys-13* and *lys-19* transcribed under the control of the P22 P_{R} promoter sequence. Since arabinose is not present in animal tissues, C2 protein would cease to be synthesized when the vaccine strain was in vivo and gradually permit derepression of genes such as lethal genes repressed by the C2 protein. This would constitute a delayed death after the vaccine strain entered the immunized animal host which would be considered a non-permissive environment because of the absence of arabinose but the vaccine strain would survive long enough to reach lymphoid tissues and induce an immune response. It should be noted that arabinose binds to the *araC* gene product which then serves as an activator for transcription from the P_{BAD} promoter to enable transcription of *c2*. In the absence of arabinose, the AraC protein serves as a repressor to block transcription from the P_{BAD} promoter with a failure to synthesize C2. In this particular construction, transcription terminators were placed on either side of the insertion within the *asd* defined deletion. The suicide vector pMEG-611 with the *ΔasdA19::*TT*araC*P_{BAD}*c2*TT deletion/insertion mutation is depicted in Figure 5. The suicide vector can be electroporated or transformed into the suicide vector donor MGN-617 and this strain used for conjugational transfer with a suitable recipient strain such as *S. typhimurium* UK-1 wild-type χ3761. In this case ampicillin and chloramphenicol resistance can be used to select for the single crossover event causing the suicide vector to integrate into the chromosome making use of sequences flanking the *Δasd* mutation for homologous reciprocal crossover recombination. Following selection for ampicillin and chloramphenicol resistant isolates, small cultures can be grown and then plated on medium with 5% sucrose and DAP. In some cases the second crossover will occur on the opposite side of the *asd* deletion from the site of the first crossover event and result in replacement of the chromosomal wild-type *asd*⁺ allele with the *ΔasdA19*::TT*araC*P_{BAD}*c2*TT construction. The *S. typhimurium* UK-1 strain MGN-798 was constructed in this manner.

We next introduced various Asd⁺ vectors into MGN-023 with the *ΔasdA16* mutation, MGN-392 with the *ΔasdA17*::TT*c1857λ*P_{R}P22*c2* mutation and into MGN-798 with the *ΔasdA19*::TT*araC*P_{BAD}*c2*TT mutation. In these studies we were interested in determining comparative growth of strains, their comparative ability to colonize lymphoid tissues in orally immunized mice, and importantly to cause disease. In this regard, it would be expected that the expression of the wild-type *asd* gene on a plasmid should theoretically restore full virulence to a strain which should now grow independently of the presence of DAP and exhibit properties more or less like the *S. typhimurium* UK-1 wild-type parent χ3761. As a control for these experiments, MGN-392 was transduced to Asd⁺ using phage P22 propagated on χ3761 to yield strain MGN-491. In the first experiment, which was as a survey, pMEG-086 (Table 1B) was introduced into both MGN-023 and MGN-392. pMEG-086 has a pUC origin of replication and we have observed that over expression of the *asd* gene product can reduce virulence. We therefore also introduced pYA810 (Table 1B) into MGN-392 since pYA810 has a p15A origin of replication resulting in a much lower plasmid copy number and thus produces a much lower, but sufficient, level of Asd enzyme. We also introduced the Asd⁺ containment vector pMEG-105 into MGN-392 which was grown at 37°C or above. (It should be noted that the presence of the containment vector pMEG-105 will kill bacteria that are unable to express the P22 *c2* gene so that the expression of the plasmid encoded *lys*-*13* and *lys-19* genes is repressed by C2 protein.) All of these strains grew with more or less equivalent rates of growth in Luria broth in the absence of exogenous DAP. When DAP was added to the media and strains were repetitively transferred over a period of 50 generations of growth, all isolates, approximately 100 isolates of each strain, were Asd⁺ indicating stable maintenance of each of the plasmids in the various genetic backgrounds.

We next took the strains, grew them at 37° C in Luria broth to an OD₆₀₀ of 0.8, except for one subculture of the MGN-392 (pMEG-105) culture which was shifted to 42°C for two hours prior to harvesting for use to orally immunize mice. Eight week old female BALB/c mice were held in quarantine for one week prior to use in experiments, deprived of food and water for approximately four hours prior to receiving doses of vaccine administered in a 20 µl volume behind the incisors using a micropipette. Food and water were returned thirty minutes after oral immunization and the mice were observed over a thirty day period.

As revealed by the data in Table 2, all mice inoculated with MGN-491, the Asd⁺ transductant of MGN-392, died as did both mice infected with MGN-023 harboring pMEG-086. In contrast, under no circumstance did any of the mice infected with derivatives of MGN-392 succumb to infection, indicating that the *asd*⁺ gene on the plasmid vector was unable to complement the effect of the *ΔasdA17*::TT *c1857*λP_{R}P22*c2* deletion/insertion mutation. Since the extent of the *asd* deletion is identical in the *ΔasdA16* and the *ΔasdA17* mutations, the enhanced avirulence of the MGN-392 strain with various Asd⁺ vector constructs must be due to some effect of the insertion. Since the P22 *c2* gene is transcribed from the λP_{R} in a direction out of the deleted *asd* gene, it must be construed that very strong transcription must interfere with expression of genes adjacent to the deleted *asd* mutation and contribute in some unknown manner to the attenuation of such strains.

To test this hypothesis and explore this phenomenon more fully, a more extensive series of animal experiments were conducted, but this time also making use of strain MGN-795 with the Δ*asdA19*::TT*araC*P_{BAD}*c2*TT deletion insertion. In this case, transcription terminators are placed at the end of the transcribed *araC* gene and also 3' to the transcribed *c2* gene to see whether that would eliminate attenuation due to the insertion. In these studies we made use of pYA292 or pYA810 (Table 1B)which are Asd⁺ vectors with the p15A origin of replication. The wild-type strain χ3761 had an oral LD₅₀ of 5.8 x10⁴ (Table 3). On the other hand, MGN-023 in the absence of an Asd⁺ vector was totally avirulent and had an LD₅₀ in excess of 6.8 x10⁸ CFU. In conformance with the results in Table 3, MGN-392, when endowed with pYA810, was unable to kill any mice, even when administered at very high doses, and thus has an LD₅₀ in excess of 7.6 x10⁸ CFU. In contrast, MGN-023, whether endowed with the Asd⁺ vector pYA292 or pYA810, gave an LD₅₀ only slightly higher than for the wild-type parent, a result importantly analogous to the result for strain MGN-795 that possesses the *ΔasdA19*::TT*araC*P_{BAD}*c2*TT insertion complemented by pYA810. It is therefore evident from these data that the inclusion of transcription terminators on both ends of an insertion within the *asd* gene precludes the inserted sequences from interfering with expression of adjacent genes which might hyperattenuate the vaccine strain and thus reduce immunogenicity.

**TABLE 2**

| Mortality of S-week old BALB/c mice 30 days after oral inoculation with *S*. *typhimurium* UK-1 strains MGN-023 ( with Δ *asdA16*) and MGN-392 (with *ΔasdA17*::TT*cI857* P_{R} *c2*) containing Asd⁺ vectors pYA810 (p15A ori), pMEG-086 (pUC ori) or the containment vector pMEG-105 (p15Aori) in comparison to the Asd⁺ transductant strain MGN-491 (derived from MGN-392). | | | |
|---|---|---|---|
| Strain | Genotype^{c} | Inoculating dose (CFU) | Survivors/total |
| MGN-491 | Asd⁺ | 1.8 x 10⁷ | 0/2 |
| | | | |
| MGN-023 (pMEG-086) | *ΔasdA16* (Asd⁺ vector with pUCori) | 8.4 x 10⁶ | 0/2 |
| | | | |
| MGN-392 (pYA810) | *ΔasdA17*::TT*cI857* P_{R} *c2* (Asd⁺ vector with p15Aori) | 7.5 x 10⁶ | 2/2 |
| | | | |
| MGN-392 (pMEG-086) | *ΔasdA17*::TT*cI857* P_{R} c2 (Asd⁺ vector with pUCori) | 2.7 x 10⁶ | 2/2 |
| | | | |
| MGN-392^{a} (pMEG-105) | *ΔasdA17*::TT*cI857* P_{R} *c2* (containment Asd⁺ vector) | 1.0 x 10⁷ | 2/2 |
| | | | |
| MGN-392^{b} (pMEG-105) | *ΔasdA17*::TT*cI857* P_{R} *c2* (containment Asd⁺ vector) | 4.4 x 10⁶ | 2/2 |

| | | | |
|---|---|---|---|
| ^{a} strain grown at 37°C under conditions in which lysis of strain is minimal (less than several percent). | | | |
| ^{b} strain grown at 42°C for one hour prior to infection so that phage lysis genes on containment vectors are totally repressed and there is no cell lysis. | | | |
| ^{c} TT as shown in the various constructs is *rrnB*T1T2 | | | |

**TABLE 3**

| Mortality of 8-week-old BALB/c mice 30 days after oral inoculation with *S*. *typhimurium* UK-1 wild-type χ3761 and MGN-023 (*ΔasdA16*), MGN-392 (*ΔasdA17*::TT*cI857* P_{R} *c2),* and MGN-795 (*ΔasdA19*::TT*araC*P_{BAD}*c2*TT) containing an Asd⁺ vector (pYA292 or pYA810) with the p15A origin of replication. | | | | |
|---|---|---|---|---|
| **Strain** | **Genotype** | **Inoculating dose (CFU)** | **Survivors/total** | **LD**_{**50**} **(CFU)** |
| | | | | |
| χ3761 | wild-type | 1 x 10⁷ | 0/2 | 5.8 x 10⁴ |
| | | 1 x 10⁶ | 1/5 | |
| | | 1 x 10⁵ | 1/5 | |
| | | | | |
| MGN-023 | *ΔasdA16* | 6.8 x 10⁸ | 5/5 | >6.8 x 10⁸ |
| | | | | |
| MGN-023 | *ΔasdA16* (Asd⁺ | 6.6 x 10⁶ | 0/5 | 2.1 x 10⁵ |
| (pYA292) | vector with | 6.6 x 10⁵ | 0/5 | |
| | p15A ori) | 6.6 x 10⁴ | 5/5 | |
| | | | | |
| MGN-023 | *ΔasdA16* (Asd⁺ | 7.6 x 10⁶ | 2/5 | 4.2 x 10⁵ |
| (pYA810) | vector with | 7.6 x 10⁵ | 1/5 | |
| | p15A ori) | 7.6 x 10⁴ | 4/5 | |
| | | 7.6 x 10³ | 5/5 | |
| | | | | |
| MGN-392 | *ΔasdA17*::TT | 7.6 x 10⁸ | 5/5 | >7.6 x 10⁸ |
| (pYA810) | *cI857* P_{R} *c2* | 7.6 x 10⁷ | 5/5 | |
| | (Asd⁺ vector | 7.6 x 10⁶ | 5/5 | |
| | with p15A ori) | 7.6 x 10⁵ | 5/5 | |
| | | 7.6 x 10⁴ | 5/5 | |
| | | | | |
| MGN-795 | *ΔasdA19*::TT | 6.4 x 10⁶ | 0/5 | 1.3 x 10⁵ |
| (pYA810) | *araC*P_{BAD}*c2*TT | 6.4 x 10⁵ | 1/5 | |
| | (Asd⁺ vector | 6.4 x 10⁴ | 3/5 | |
| | with p15A ori) | 6.4 x 10³ | 5/5 | |

### EXAMPLE 2

This example illustrates the construction of defined deletions in the phoP gene.

### Introduction

*Salmonella* mutants with mutations in the regulatory *phoP* locus are attenuated and immunogenic, providing protective immunity to challenge with virulent *Salmonella* strains (Galan and Curtiss, *Microbial Pathogenesis 6*:433-443, 1989; Miller et al., *Proc. Natl*. *Acad. Sci. 86*:5054-5058, 1989). These microbes can also be used as effective antigen delivery vectors to elicit strong immune responses to expressed foreign proteins (Wick et al., *Mol. Microbiol*. *16*:465-476, 1995). Kier et al. (*J*. *Bacteriol. 138*:155-161, 1979) conducted a genetic analysis of the ability of *S*. *typhimurium* to produce a non-specific acid phosphatase and identified mutations in two distinct genetic loci which eliminated the ability of *Salmonella* to produce acid phosphatase. One gene termed *phoN* was linked to the *purA* gene and another designated *phoP* was linked to the *purB* gene in a different part of the bacterial chromosome. Because of the ability to isolate temperature-sensitive alleles mapping at the *phoN* locus that caused acid phosphatase activity to be heat sensitive and their ability to isolate mutations closely linked to the *phoP* locus which rendered synthesis of non-specific acid phosphatase constitutive, they were led to the belief that the *phoN* gene constituted the structural gene for the non-specific acid phosphatase and that the *phoP* gene specified a regulatory function governing *phoN* expression.

In 1986, Fields et al. (*Proc. Natl*. *Acad. Sci. 83*:5189-5193) reported on the isolation of a number of *S. typhimurium* mutants with mutations that rendered *Salmonella* sensitive to killing by murine macrophages. In 1989, Fields et al. *(Science 243*:1059-1062) discovered that several of their macrophage-sensitive mutants possessed mutations in the *phoP* gene, also inactivating the ability to produce non-specific acid phosphatase. Although Fields et al. (*supra*, 1989) reported the avirulence of these mutants, they found no evidence of their immugenicity and, indeed, concluded that they were non-immunogenic. Galan and Curtiss (*supra*) demonstrated that mouse virulent *S. typhimurium* strains possessing the *phoQ12* mutation (formerly referred to as *phoP12* mutation), isolated and carefully characterized by Kier et al. (*supra*) were totally avirulent when used to orally infect mice at high doses, but found, in contrast to Fields et al., that these mice displayed a state of immunity conferring high-level protection to these mice when challenged with 10,000 times a lethal dose of the wild-type virulent *S. typhimurium* parent. Miller et al. (*supra*) and Groisman et al. (*J. Bacteriol*. *174*:486-491, 1989) both showed that the *phoP* locus was in reality an operon containing two genes which constituted a two-component regulatory system. Miller et al. termed the genes *phoP* and *phoQ*. Groisman initially designated them *phoP* and *phoZ*, but in a footnote to their 1989 paper renamed *phoZ* as *phoQ* in conformance with the choice of Miller et al. A map of the *phoPQ* operon with the linked *potA*, *pepT* and *purB* genes is shown in the Miller et al reference. The *phoQ* gene is a sensor kinase that responds to the concentration of magnesium (Mg²⁺), and to a lesser extent calcium (Ca²⁺), to phosphorylate the *phoP* gene product (Garcia Vescovia et al., *J*. *Biol*. *Chem*. *272*:1440-1443, 1997) which serves as a DNA binding regulatory protein to activate expression of genes termed *phoP*-activated genes *(pag)* or to serve as a repressor for *phoP*-repressed genes *(prg)* (Miller et al., 1989 *supra*; Miller, *Mol*. *Microbiol*. *5*:2073-2078, 1991). It is now known that the *phoPQ* two-component regulatory system influences many attributes of *Salmonella*, including invasion in the intestinal tract, tolerance to acid stress, resistance to polypeptide defensins, survival in macrophages, structure and composition of lipopolysaccharide (LPS) components, and for the overall invasiveness and virulence of *Salmonella* in animals and humans.

### Construction of phoP defined deletions

A 2,110 base pair *Bam*HI to *Cla*I fragment was PCR amplified from the *S. typhimurium* UK-1 strain χ3716, which fragment contained all of the *phoPQ* operon including 5' and 3' regulatory sequences, and inserted into the SC101 cloning vector pWKS30 (Table 1B) that had similarly been digested with *Cla*I and *Bam*HI. This plasmid, when introduced into *a phoP S*.*typhimurium* mutant such as MGN-069 (Table 1), complements the PhoP⁻ phenotype and enables the strain to synthesize the *phoN* encoded non-specific acid phosphatase and to exhibit other properties of a PhoP⁺ *S. typhimurium* strain.

Many of the mutants with mutations conferring a PhoP⁻ phenotype, whether induced with chemical mutagens or transposon insertional mutagenesis, have been found to have little or no *phoQ* gene activity either because the mutation was in the *phoQ* gene or the mutation even though being in the *phoP* gene, had a polar effect on preventing *phoQ* gene expression. For this reason, we constructed the *ΔphoP22* deletion which is a defined deletion mutation of the *phoP* gene that permits wild-type expression of the *phoQ* gene. This was accomplished by digesting pMEG-063 (Fig. 18) DNA having the *phoPQ* operon with *Eco*RV which removes 514 base pairs within the *phoP* gene to result, after religation, in pMEG-068. The *ΔphoP22* deletion so generated results in a 514 base pair deletion with a 47 amino acid sequence from the N-terminal end of PhoP fused to an out of frame 8 amino acid sequence whose coding sequence overlaps by 5 base pairs the N-terminal beginning of the *phoQ* gene sequence. Aspartic acid at amino acid 52 within the PhoP sequence is deleted. It is this aspartate that is phosphorylated by the sensor kinase present in the *phoQ* gene encoded protein. The construct is so made so that if there is a ribosomal binding sequence encoded in the 3' end of the *phoP* gene proceeding the start of the *phoQ* gene, the sequence is still retained in the deletion mutation specified by the sequence.

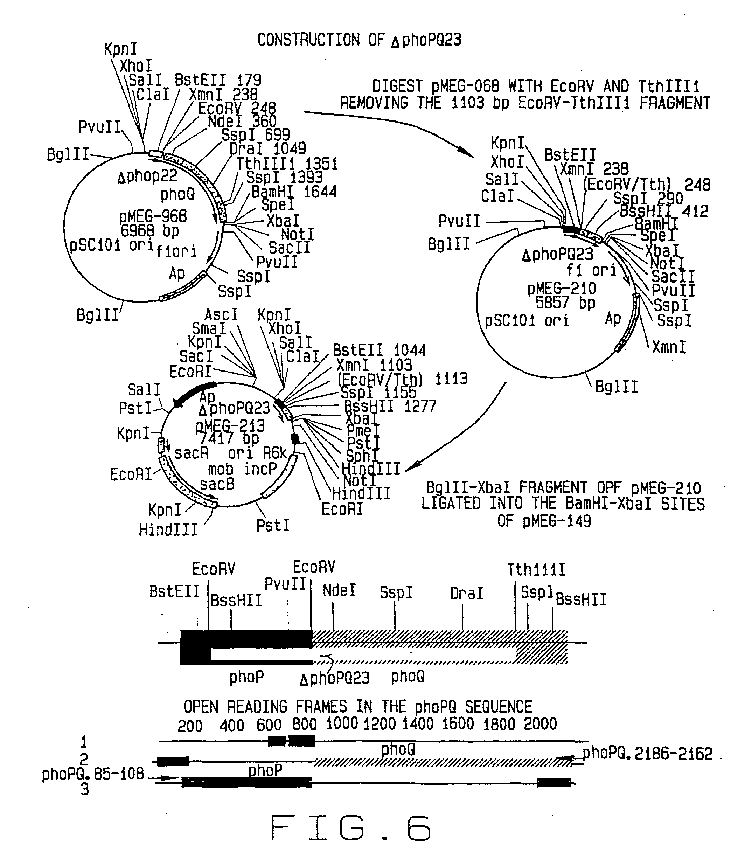

The construction of *ΔphoPQ23* which illustrates the construction of *phoPQ* defined deletions is shown in Figure 6. To generate the *ΔphoPQ23* defined deletion mutation, pMEG-068 was digested with *Eco*RV, at the restriction site restored when the *ΔphoP22* deletion was generated and the restriction enzyme *Tth*111I to remove an additional 1,103 base pair fragment encoding the C-terminal end of the *phoP* gene and up to the last 90 amino acids of the *phoQ* gene. This deletion specifically deleted the histidine-259 residue that is phosphorylated in the PhoQ protein and serves as the phosphate donor for phosphorylation of the aspartate-52 in the *phoP* gene. pMEG-210 containing the *ΔphoPQ23* deletion was digested with *Bgl*II and *Xba*I and this fragment ligated into the suicide vector pMEG-149, DNA that had similarly been digested with *Bam*HI and *Xba*I. This resulted in the suicide vector pMEG-212 which was electroporated into the suicide vector donor strain MGN-617. Conjugational mating of MGN-617 (pMEG-212) with various *S*. *typhimurium* strains as described above could be used to initially isolate single crossover events and then double crossover events in which the *ΔphoPQ23* mutation had replaced the wild-type *phoPQ* alleles resulting in a derivative unable to exhibit the blue color by non-specific acid phosphatase digestion of the chromogenic substrate BCIP incorporated into the agar medium.

A defined deletion mutation within the *phoQ* gene was generated in a similar way. The plasmid pMEG-198 which contains the *phoQ* gene and flanking sequences generated by subcloning from pMEG-063, was digested with *Nae*I and *Dra*I. The DNA treated with the Klenow fragment to fill in gaps and religated. This removed a 716 base pair sequence within the *phoQ* gene, including that encoding the histidine-259 that is normally phosphorylated, to yield pMEG-202. pMEG-202 was digested with *Xba*I and *Xho*I and the fragment containing the *ΔphoQ*1 mutation was cloned into the suicide vector pKNG-101, similarly digested with *Xba*I and *Xho*I. The resulting suicide vector was electroporated into the suicide vector donor in strain MGN-617. As above, this strain could be mated with various *S. typhimurium* strains, single crossovers selected by streptomycin resistance, and the double crossover as resistant to 5% sucrose. The success of the double crossover event was again revealed by the incorporation of BCIP in the agar medium and scoring for white colonies unable to hydrolyse the phosphatase substrate.

Strains were constructed with *ΔphoP22*, the *ΔphoQ1*, and *ΔphoPQ23* deletion mutations. Strains with the *ΔphoP22* mutation had a deletion of approximately 514 base pairs, strains with the *ΔphoPQ23* deletion lacked approximately 1,617 base pairs, and strains with the *ΔphoQ1* mutation a deletion of approximately 716 base pairs.

### EXAMPLE 3

This Example illustrates the construction of defined deletions in the *phoPQ* operon with insertion of a constitutively expressed reporter gene cassette which includes a transcription terminator.

For both in vitro and in vivo studies to evaluate complementation of specific mutational lesions, and to verify the existence of both the wild-type allele present on a low copy number plasmid and the mutant allele in the chromosome, we have often inserted the *xylE* reporter gene (Kanega et al, Molecular Microbiol 13:555-568, 1994) into the chromosomal mutant gene. Colonies with bacteria that express the *xylE* gene will turn yellow in about five minutes after being sprayed with a 250 mM catechol solution and the spray is without effect on the viability of cells within the colony. When we used a *xylE* structural gene cassette without a promoter and depended upon expression from the promoter used for expression of the gene into which the *xylE* cassette was inserted, expression could be strong to non-existent dependent upon how the particular gene into which the *xylE* cassette was inserted was regulated in response to environmental stimuli. We therefore fused the *xylE* gene to the bacteriaphage T4 gene 32 promoter which permitted constitutive expression of the *xylE* gene product under a diversity of conditions.

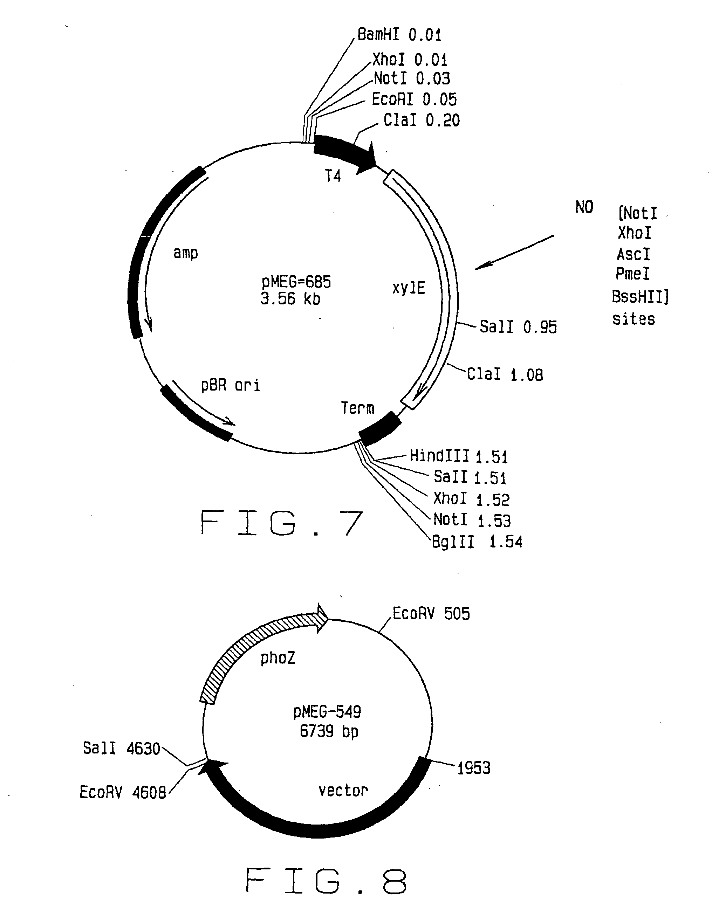

Unfortunately, because of the strength of the gene 32 promoter, RNA transcription could proceed beyond the bounds of the insertion either in a direction that would affect expression positively or negatively of adjacent genes depending upon whether the mRNA represented a sense or anti sense strand. To circumvent this problem, a strong transcription terminator was placed after the C-terminal end of the *xylE* gene. Figure 7 depicts the pMEG-685 plasmid from which the gene 32 promoter *xylE* gene with the gene 32 transcription terminator can be cloned as a cassette using various restriction enzymes and inserted in place of a deleted segment from any gene.

To make a suicide vector with the T4 gene *32* promoter *xylE* transcription terminator (P₃₂*xylE*TT) cassette in the *phoQ* gene, the following steps were performed. pMEG-063 (Fig. 18), which possesses the *phoPQ* operon, was digested with *Kpn*I, treated with the Klenow fragment of DNA polymerase to create blunt ends, and then digested with *Xba*I to generate a 2.4kb *phoPQ* fragment to be ligated to pMEG-197 digested with *Dra*I and *Xba*I. pMEG-197 is an Asd⁺ vector which possesses a deletion of the *trc* promoter (P_{TRC}) which was achieved by deleting a *Bgl*II-*Bam*HI fragment of pMEG-195 and relegating to generate pMEG-197. The absence of P_{TRC} would therefore facilitate subsequent cloning events so that over expression of *phoPQ* gene products would not be toxic to bacterial cells. The resulting plasmid, pMEG-198, was digested with *Nae*I and *Dra*I to remove a 716 bp segment of the *phoQ* gene. At the same time, pMEG-685 was digested with *Eco*RI and *Hin*dIII and the fragment treated with the Klenow fragment of DNA polymerase to generate blunt ends. This 2.4kb fragment so generated from pMEG-685 was then ligated to the pMEG-198 vector that had been digested with *Nae*I and *Dra*I. The resulting plasmid generated after ligation was designated pMEG-201. pMEG-201 now contains the T4P₃₂*xylE*TT cassette within the *phoQ* gene in place of the deleted 716 bp of the *phoQ* gene. pMEG-201 was now digested with *Xba*I and *Xho*I to generate a 4.0 kb fragment that was inserted into *Xba*I-*Xho*I digested suicide vector pKNG-101 to generate the recombinant suicide plasmid pMEG-205 which was introduced into the suicide vector donor MGN-617. This strain, MGN-740, could be used to transfer the suicide vector into suitable *Salmonella* recipients such as *S.* *typhimurium* UK-1 χ3761 or *S*. *typhimurium* SL1344 χ3339 selecting initially for streptomycin resistance for the single crossovers and then sucrose resistance to select for loss of the suicide vector and the desired double crossover. Success in allele replacement can be detected as white colonies when plated on agar media overlaid with a soft agar layer containing the α-naphthylphosphate BCIP substrate for non-specific acid phosphatase in a pH 5.5 acetate buffer as described by Kier et al (1979, *supra*). All colonies that failed to exhibit acid phosphatase activity were able to display expression of *xylE* and hydrolyze catechol to generate a yellow pigment. Strains such as MGN-748 (Table 1A) possessing the *ΔphoQ2*::T4P₃₂*xylE*TT insertion were totally avirulent when evaluated in mice. In the presence of a plasmid like pMEG-063, for example, MGN-748 would now hydrolyze BCIP demonstrating the ability to synthesize *phoN* specified acid phosphatase, but also turned yellow when sprayed with catechol, showing the retained presence of the *xylE* insertion within the deleted *phoQ* gene. Such strains had wild-type virulence when evaluated in mice, showing that the complementation was effective. In this regard, reisolation of bacteria from infected mice five to seven days after infection yielded bacteria that still possessed pMEG-063, still produced acid phosphatase, and still expressed the *xylE* gene. Thus the inclusion of the promoter-driven *xylE* gene followed by a transcription terminator did not have an adverse effect in precluding complementation of the defect from a plasmid possessing either the *phoPQ* operon or in another test construct which only expressed *phoQ* because of deletion of the *phoP* gene as present in a plasmid such as pMEG-068 which expresses *phoQ* but lacks the *phoP* gene sequence due to the *ΔphoP22* mutation. This experiment also validated that the *ΔphoP22* deletion mutation did not interfere with the normal expression of the downstream *phoQ* gene.

### EXAMPLE 4

This example illustrates the construction of deletion mutation, *ΔphoP1918* in which the entire coding region of the *phoP* gene along with promoter region and terminal nucleotide of the 5' adjacent gene is deleted, in absence of the insertion of a transcription terminator in the deleted gene site.

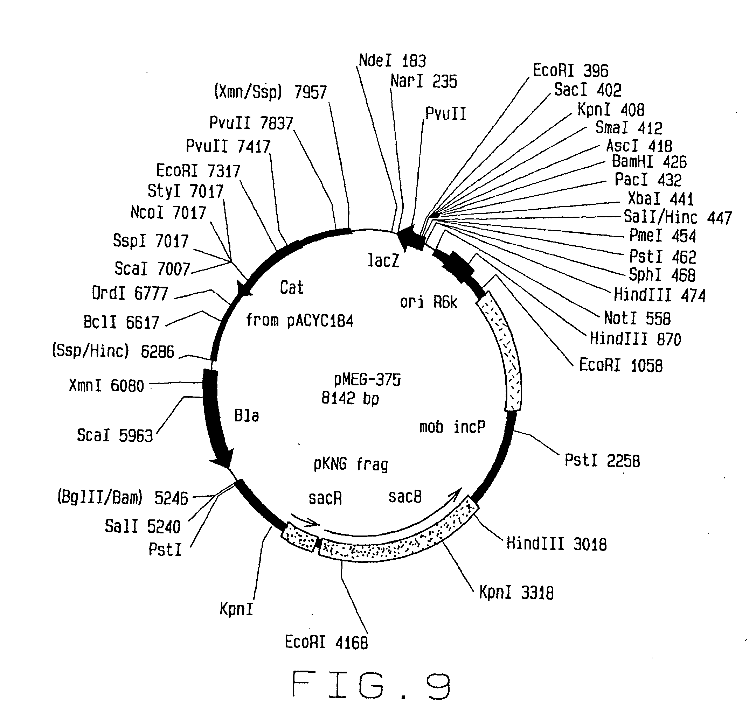

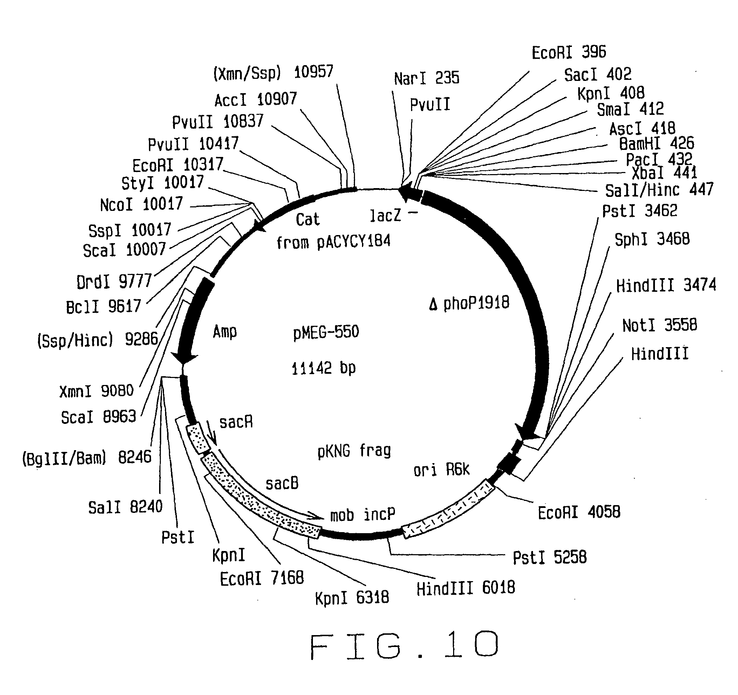

The defined deletion, termed Δ*phoP1918*, was designed to delete the entire *phoP* gene and its promoter regulatory sequences. Primers were designed based upon the published sequence of the *phoPQ* gene sequence (Miller et al, 1989, *supra*) as follows: The forward primer was derived from the 5' end of *phoQ* gene; the reverse primer consisted of *phoP* upstream sequences beyond the -10 and -35 regions; the ends of these primers contained the endonuclease *Not*I recognition sequence (GGCGCGCC) (SEQ ID NO:23) for ligation and cloning. The forward primer contained the sequence (5' to 3') ttggcgcgcc TG AAT AAA TTT GCT CGC CAT TTT CTG (SEQ ID NO:24) and the reverse primer contained the sequence (5' to 3') ttggcgcgcc TA AAC CAG ACA AAT AGT CAC CC (SEQ ID NO:25). Small letters denote non*-phoPQ* sequences including the *Not*I endonuclease recognition sequence. Plasmid pEG-5381, described by Groisman et al. (*Proc. Nat*. *Acad. Sci. USA 86:* 7077-7081, 1989), was used as a base plasmid to generate the *phoP1918* deletion by inverse polymerase chain reaction (Inverse-PCR) using forward and reverse primers described above. Note that Groisman et al. termed the *phoQ* gene as *phoZ* but agreed to subsequently use of the *Q* designation in a footnote to their paper. The PCR product was digested with restriction endonuclease *Not*I, ligated and transformed into *E*.*coli* strain MGN-026. The resultant strain, MGN-2052, contained the plasmid, pMEG-549 (Fig. 8), with the *phoP1918* deletion as described above. Plasmid pMEG-549 has two recognition sites for endonuclease *Eco*RV and digestion of pMEG-549 with *Eco*RV will remove a DNA fragment with the *ΔphoP1918* mutation. This *Eco*RV DNA fragment was inserted into the *Pme*I site in the suicide plasmid pMEG-375 (Fig. 9) by ligation to yield pMEG-550 (Fig. 10) which was transformed into the *E.coli* suicide vector donor strain MGN-617. MGN-617 (pMBG-550), was resistant to ampicillin and chloramphenicol at 100 µg/ml and 25 µg/ml, respectively.

Strain MGN-617 (pMEG-550) was used to conjugate with *S*. *typhimurium* strains χ3761 (UK-1) and χ3339 (SL1344) (Table 1A). Ex-conjugants were selected on Luria agar with chloramphenicol. Individual colonies were checked for resistance to ampicillin and chloramphenicol and cells from some of these colonies were picked, grown as small cultures which were plated on Luria agar (without salt) containing 5% sucrose and incubated at 30°C. Sucrose-resistant colonies were selected and screened for both ampicillin and chloramphenicol sensitivities. These colonies were further screened for absence of acid phosphatase (white colonies as opposed to red wild-type colonies) using the acid phosphatase over-lay test (Kier et al, 1979). Some of the white colonies that lacked acid phosphatase activity were further subjected to serotyping for group B *Salmonella* and smooth LPS profile on SDS-gels. The *ΔphoP1918* mutations in the chromosome of these mutant *Salmonella* strains were checked by PCR. The mutant strains were designated MGN-2083 to MGN-2086 (Table 1).

The sequence of the *ΔphoP1918* mutation is as follows. The first 70 bp are identical to those in the nucleotide sequence of the *phoPQ* region sequenced by Miller et al., (1989). The next 8 base pairs constitute the *NotI* recognition sequence GGCGCGCC (SEQ ID NO:23) with the C at bp 78 being a base pair substitution for the A which starts the coding sequence of the *phoQ* gene in wild-type *S. typhimurium*. Since the initiation codon for proteins is ATG 91% of the time, GTG 8% of the time, TTG only 1% of the time and CTG essentially never, it can be expected that the *phoQ* gene product will seldom be synthesized in strains with the *ΔphoP1918* mutation. There are, however, several internal methionines specified by ATG codons at bp 1052,1067,1277, 1322,1382 and 1526 (all before the histidine codon at bp 1643 which specifies the phosphorylated histidine) and which could possibly lead to production of a gene translation product.

Although the deletion of the *phoP* gene in its entirety and base pair substitution for the first base of the *phoQ* gene should result in strains defective for expression of either the *phoP* or *phoQ* gene product, the deletion also deletes sequences upstream from the *phoP* gene that not only would delete the -35 and -10 promoter sequences, but might interfere with transcription termination at the end of the *purB* gene which is transcribed in the same direction as *phoP* and *phoQ*. Thus under some circumstances, under limiting purine concentration which is the situation in vivo, the *purB* gene would be expressed at a high level. It is conceivable that the transcript would continue after the *purB* gene leading to transcription across the deletion and into the coding sequence for the adjacent gene pepT which encodes a peptidase. However, the *pepT* gene is transcribed in the opposite direction of *purB* and *phoPQ* so that any transcript continuing beyond the mutation in the *phoPQ* operon would make an anti-sense RNA that could then inhibit the expression of the pepT gene by hybridizing to pepT mRNA and thus blocks its translation. Since it is not known whether this peptidase plays an important role in vitro or in vivo, an improved way of generating a mutation such as caused by the *ΔphoP1918* mutation, would be to insert within the deletion a transcription terminator such as *rrnB*T1T2 or the *trpA*TT (Figure 1) which would preclude transcription across the mutational defect.

### EXAMPLE 5

This example illustrates the construction of strains with the *ΔphoP24* mutation and an insertion sequence containing the *trpA* transcription terminator.

The defined *ΔphoP24* mutation was designed to include insertion of a transcription terminator and this was introduced into *S. typhimurium* SL1344 to produce strain MGN-1362.

The *ΔphoP24* deletion was obtained using inverse PCR with primers *phoP* 40-20 and *phoP* 815-839 based on the Miller et al (1989, *supra*) sequence of the *phoPQ* region to remove the entire coding region of *phoP* and 100 bp of upstream DNA from the *phoPQ* clone pEG-5381 (Groisman et al., *Proc. Nat*. *Acad. Sci. USA 86*: 7077-7081, 1989). Although this deletion removes all of the *phoP* coding region, the *phoQ* coding region is left intact. This PCR product was then digested with *Bgl*II and *Eco*RI to cut within the designed primers and provide compatible ends for ligation to the annealed *trpA* transcription terminator oligos (Fig. 11). The ligation described resulted in the production of pMEG-359 (Fig. 12). The 3.1 kb *Eco*RV fragment of pMEG-359 was then cloned into the *Sma*I site of the suicide vector, pMEG-149, to produce the *ΔphoP24* suicide plasmid pMEG-368 (Fig. 13). The deletion of the *phoP* region and insertion of the *trpA* transcription terminator was then confirmed by sequence analysis. Since pMEG-368 is a mobilizable suicide vector encoding for the selectable ampicillin resistance marker and the counter selectable marker, levansucrase, resulting in sensitivity to sucrose, the plasmid can be conjugated into any strain desired selecting for ampicillin resistance followed by counter-selection for the replacement of the wild-type *phoP* gene with the *ΔphoP24* mutation in the presence of sucrose. The strain responsible for the delivery of pMEG-368 was obtained by electroporating pMEG-368 into the Pir⁺ Asd⁻ delivery host MGN-617 to produce MGN-617 (pMEG-368). The pMEG-368 suicide construct was then conjugationally transferred into the *S. typhimurium* SL1344 strain χ3339, followed by selection for ampicillin resistant isolates which grew without DAP (diaminopimelic acid). One of the isolates, MGN-1361, from this conjunction, representing the single integration of the *ΔphoP24* deletion plasmid into the chromosome to produce a duplication of the *phoP* region with both the wild-type *phoP* gene and the mutant *ΔphoP24* allele. MGN-1361 was then plated on Luria agar containing 5% sucrose to select for loss of the ampicillin-resistance suicide vector. The isolates obtained by this selection were then screened for acid phosphatase activity using the agar overlay method of Kier et al. (*J*. *Bacteriol. 130*:399-410, 1977). The white phosphatase-negative colonies were then confirmed for the *ΔphoP24* phosphatase minus phenotype and stocked as MGN-1362 (Table 1A).

We have further used strain MGN-617 (pMEG-368) to introduce the *ΔphoP24* mutation into a diversity of *S*. *typhimurium*, *S. paratyphi A* and *S. typhi* strains. In all cases the strains failed to express the non-specific acid phosphatase encoded by the *phoN* gene and using PCR analysis had approximately 730 base pairs of DNA less than in the *phoPQ* wild-type strain. This represented a deletion of 775 bp of the *phoP* gene and the insertion of 45 bp specifying the *trpA* transcription terminator.

### EXAMPLE 6

This example illustrates the evaluation of *S. typhimurium* mutants with defined deletion mutations in the *phoPQ* operon for phenotypic properties, growth rate, colonizing ability in mice, and avirulence and immunogenicity in mice.

*S. typhimurium* SL1344 (χ3339) and UK-1 (χ3761) mutants with the defined deletion mutations in the *phoPQ* operon as described above are listed in Table 4.

**TABLE 4**

| *S. typhimurium* SL1344 (χ3339) and UK-1 (χ3761) mutants with defined deletion mutations in the *phoPQ* operon. | | |
|---|---|---|
| Defined Δ mutation | SL1344 strain | UK-1 strain |
| | | |
| *ΔphoP22* | χ8299 | χ8087 |
| | | |
| *ΔphoQ1* | χ8430 | χ8088 |
| | | |
| *ΔphoPQ23* | χ8297 | χ8089 |
| | | |
| *ΔphoP24* | χ8597 | χ8429 |
| | | |
| *ΔphoP1918* | χ8432 | χ8431 |

All of these strains were subjected to phenotypic characterization using API test strips, evaluated for susceptibility to a diversity of antibiotics using zones of inhibition by placing antibiotic impregnated discs on a soft agar overlay containing each of the ten mutant strains and their two parents using Penassay agar base. All of the strains had an API profile ID of 6704552 which is a very good identification for *Salmonella enterica* serotypes. All the strains were susceptible to all of the antibiotics tested except for rifampin (5 µg dose) and for the χ3339 SL1344 derivatives which all displayed resistance to streptomycin which is characteristic of the SL1344 parent (Table 1). All strains were motile, expressed Type 1 fimbriae as revealed by yeast hemagglutination that was inhibitable by mannose and had a smooth LPS phenotype. Using the soft agar overlay assay for non-specific acid phosphatase described by Kier et al (1977, *supra*), all of the mutants did not synthesize non-specific acid phosphatase. Mutant derivatives of the strains listed in Table 4 that express non-specific acid phosphatase can be isolated at frequencies of 10⁻⁷ to 10⁻⁹ with these frequencies increased by mutagenic treatment of cultures. The expression of acid phosphatase has no effect on the avirulence and immunogenicity of *phoP* mutants and *phoN* mutants have wild-type virulence for mice. It is thus apparent that these *phoN* expressing mutants have a mutation that causes transcription of the *phoN* gene to be independent of the phosphorylated *phoP* gene product. It should also be noted that these *phoN* expressing mutants isolated in the *ΔphoP* background still display all the other attributes associated with mutation in the *phoPQ* operon such as sensitivity of strains to killing by macrophages, sensitivity to defensins, altered sensitivity to polymyxin and the like.

As revealed in Figures 14 and 15, all the *S. typhimurium* SL1344 mutants with gene defects in the *phoPQ* operon grow at more or less the same rate as the wild-type SL1344 parent χ3339. Similar results have been observed for growth rates of *S*. *typhimurium* UK-1 wild-type and *phoP* mutant strains as revealed in Figure 16.

Evaluation of the mutants for avirulence and immunogenicity was performed using eight-week-old female BALB/c mice. Mice were received and held in quarantine for seven days prior to use in experiments. The standard protocol involved removal of food and water four to six hours prior to oral immunization or challenge. Bacteria were grown in Luria broth to late log phase to an OD ₆₀₀ of about 0.8, sedimented by centrifugation, resuspended in buffered saline with gelatin in a concentrated form so 20 µl samples, containing the desired number of colony forming units (CFU), could be administered with a micropipette behind the rear incisors of the mice being immunized or challenged. Food and water were returned thirty min after immunization and/or challenge. Mice were observed for thirty days following immunization and survivors challenged and then followed for another thirty days. Any mice observed to be moribund were euthanized. The results for the S. *typhimurium* SL1344 strains are included in Table 5 and the results for five *S. typhimurium* UK-1 mutants in Table 6.

**TABLE 5**

| Avirulence and immunogenicity of *S*. *typhimurium* SL1344 mutants after oral immunization and oral challenge. | | | | |
|---|---|---|---|---|
| Strain | | Immunization CFU/Dose | Challenge*with χ3339 CFU/Dose | Survivors Following Challenge/Total # of mice |
| χ3339 | Exp. 1 | | 1.0 x 10⁵ | 2/4 |
| wild-type | | | 1.0 x 10⁴ | 4/4 |
| | | | | |
| | Exp. 2 | | 1.1 x 10⁵ | 2/4 |
| | | | 1.1 x 10⁴ | 4/4 |
| Non-immunized control | Exp. 1 | | 9.2 x 10⁷ | 0/4 |
| | | | | |
| | Exp. 2 | | 8.0 x 10⁷ | 0/4 |
| χ8299 | Exp. 1 | 1.2 x 10⁹ | 9.2 x 10⁷ | 2/4 |
| Δ*phoP22* | | 1.2 x 10⁸ | 9.2 x 10⁷ | 3/4 |
| | | | | |
| | Exp. 1 | 1.2 x 10⁹ | 1.1 x 10⁹ | 4/4 |
| | | 1.2 x 10⁸ | 1.1 x 10⁹ | 3/4 |
| | | | | |
| | Exp. 2 | 1.5 x 10⁹ | 8.0 x 10⁷ | 4/4 |
| | | 1.5 x 10⁸ | 8.0 x 10⁷ | 4/4 |
| | | | | |
| | Exp. 2 | 1.5 x 10⁹ | 1.3 x 10⁹ | 4/4 |
| | | 1.5 x 10⁸ | 1.3 x 10⁹ | 4/4 |
| χ8297 | Exp. 1 | 1.1 x 10⁹ | 9.2 x 10⁷ | 4/4 |
| *ΔphoPQ23* | | 1.1 x 10⁸ | 9.2 x 10⁷ | 4/4 |
| | | | | |
| | Exp. 1 | 1.1 x 10⁹ | 1.1 x 10⁹ | 3/4 |
| | | 1.1 x 10⁸ | 1.1 x 10⁹ | 3/4 |
| | | | | |
| | Exp. 2 | 7.4 x 10⁸ | 8.0 x 10⁷ | 3/4 |
| | | 7.4 x 10⁷ | 8.0 x 10⁷ | 1/4 |
| | | | | |
| | Exp. 2 | 7.4 x 10⁸ | 1.3 x 10⁹ | 3/4 |
| | | 7.4 x 10⁷ | 1.3 x 10⁹ | 3/4 |
| χ8298 *ΔphoP24* | Exp. 1 | 1.0 x 10⁹ 1.0 x 10⁸ | 9.2 x 10⁷ 9.2 x 10⁷ | 3/4 2/4 |
| | | | | |
| | Exp. 1 | 1.0 x 10⁹ | 1.1 x 10⁹ | 4/4 |
| | | 1.0 x10⁸ | 1.1 x 10⁹ | 2/4 |
| | Exp. 2 | 8.6 x 10⁸ | 8.0 x 10⁷ | 4/4 |
| | | 8.6 x 10⁷ | 8.0 x 10⁷ | 3/4 |
| | Exp. 2 | 8.6 x 10⁸ | 1.3 x 10⁹ | 3/4 |
| | | 8.6 x 10⁷ | 1.3 x 10⁹ | 4/4 |

| | | | | |
|---|---|---|---|---|
| *Thirty days after primary immunization. | | | | |

**TABLE 6**

| Avirulence and immunogenicity of *S. typhirnurium* UK-1 mutants after oral immunization and oral challenge. | | | | |
|---|---|---|---|---|
| Strain | Immunization CFU/Dose | Survivors Following Immunization/ Total of mice | Challenge* with χ3761 CFU/Dose | Survivors Following Challenge/Total of mice |
| χ3761 | | | 7.2 x 10⁴ | 2/4 |
| | | | 7.2 x 10³ | 4/4 |
| | | | 7.2 x 10² | 4/4 |
| χ8087 | 7.6 x 10⁸ | 4/4 | 1.0 x 10⁹ | 3/4 |
| *ΔphoP22* | 7.6 x 10⁷ | 4/4 | 1.0 x 10⁹ | 2/4 |
| χ8088 | 7.2 x 10⁸ | 3/4 | 1.0 x 10⁹ | 2/3 |
| *ΔphoQ1* | 7.2 x 10⁷ | 4/4 | 1.0 x 10⁹ | 1/4 |
| χ8089 | 8.0 x 10⁸ | 4/4 | 1.0 x 10⁹ | 1/4 |
| *ΔphoPQ23* | 8.0 x 10⁷ | 4/4 | 1.0 x 10⁹ | 0/4 |
| χ8431 | 8.0 x 10⁸ | 4/4 | 1.0 x 10⁹ | 3/4 |
| *ΔphoP1918* | 8.0 x 10⁷ | 4/4 | 1.0 x 10⁹ | 2/4 |
| χ8429 | 1.6 x 10⁹ | 4/4 | 1.0 x 10⁹ | 3/4 |
| *ΔphoP24* | 1.6 x 10⁸ | 4/4 | 1.0 x 10⁹ | 3/4 |

| | | | | |
|---|---|---|---|---|
| * Thirty days after primary immunization | | | | |

Since the number of animals used per group is small in Tables 5 and 6, there is no statistically significant difference between any of the strains with regard to avirulence or immunogenicity. Nevertheless, we have observed on a number of occasions that mice orally inoculated with high titers (10⁹ CFU) of the *ΔphoQ1* UK-1 strain χ8088 sometimes died and also that the mice surviving the immunization were not as well protected against challenge with the wild-type parent as following immunization with *ΔphoP* mutants. One should not make too much of this, however, in that the *ΔphoQ*23 SL1344 mutant was totally avirulent and highly immunogenic (Table 5), whereas the one test done with the *S. typhimurium* UK-1 *ΔphoPQ23* mutant χ8089 gave very disappointing results in terms of protective immunogenicity. Since we have done other experiments with recombinant strains having the *ΔphoQ23* allele with excellent results for immunogenicity, the results with χ8089 given in Table 6 are likely to be the exception rather than the general rule.

All references cited in this specification are hereby incorporated by reference. The discussion of the references herein is intended merely to summarize the assertions made by their authors and no admission is made that any reference constitutes prior art. Applicants reserve the right to challenge the accuracy and pertinency of the cited references.

In view of the above, it will be seen that the several advantages of the invention are achieved and other advantageous results attained.

As various changes could be made in the above methods and compositions without departing from the scope of the invention, it is intended that all matter contained in the above description and shown in the accompanying drawings shall be interpreted as illustrative and not in a limiting sense.

## Claims

1. A composition comprising a pathogenic microbe having an attenuating mutation in a chromosomal gene, said mutation comprising an insertion sequence which contains a recombinant transcription terminator, in a pharmaceutically acceptable preparation.

2. The composition according to claim 1 further comprising a recombinant gene encoding a desired gene product.

3. The composition of claim 1 or 2 wherein the transcription terminator is rrnB 5s rRNA T1T2, trpA, T4 gene 32, T4 ipIII gene, or rrfG 5S rRNA.

4. The composition according to any one of claims 1 to 3 wherein the chromosomal gene is a pab gene, a pur gene, an aro gene, asd, a dap gene, nadA, pncB, galE, pmi, fur, rpsL, ompR, htrA, hemA, cdt, cya, crp, phoP, phoQ, rfc, poxR, or galU.

5. The composition according to any one of claims 1 to 4 wherein the microbe is a Salmonella, Shigella, Escherichia or hybrid thereof.

6. The composition according to any one of claims 1 to 5 wherein the transcription terminator is inserted in the phoP gene.

7. The composition according to any one of claims 1 to 6 further comprising a deletion mutation in the coding region of said gene, in the promoter region of said gene or in both the coding region and promoter region of said gene.

8. The composition according to any one of claims 2 to 7 wherein the recombinant gene encodes a gene product from a pathogen.

9. The composition according to claim 8 wherein the pathogen is a virus, bacterium, protozoan, parasite or fungus.

10. The composition according to any one of claims 2 to 9 wherein the recombinant gene encodes a product capable of suppressing, modulating or augmenting an immune response in the individual.

11. The composition according to any one of claims 2 to 10 wherein the recombinant gene encodes an autoantigen, or an allergen to the individual.

12. The composition according to claim 11 wherein the autoantigen is a gamete specific antigen.

13. A vaccine incorporating the microbe as defined in any one of claims 1 to 12.

14. The vaccine according to claim 13 wherein the insertion sequence does not contain a recombinant promoter.

15. Use of a pathogenic microbe having an attenuating mutation comprising an insertion sequence containing a recombinant transcription terminator in a chromosomal gene and a recombinant gene encoding the desired gene product in the manufacture of a medicament for delivering a desired gene product to an individual.

16. The use according to claim 15 incorporating the features of the microbe as defined in any one of claims 2 to 12 and 14.

17. Use of a pathogenic microbe having an attenuating mutation comprising an insertion sequence containing a recombinant transcription terminator in a chromosomal gene in the manufacture of a medicament for immunizing an individual against a pathogen.

18. The use according to claim 17 incorporating the features of the microbe as defined in any one of claims 2 to 12.

19. The use according to claim 18 wherein the microbe further comprises a recombinant gene which encodes an epitope from the pathogen.

20. A method for producing a carrier microbe for delivery of a desired gene product to an individual, the method comprising generating a pathogenic microbe having a recombinant gene encoding the desired gene product and an attenuating mutation comprising an insertion sequence which contains a transcription terminator in a chromosomal gene.

21. A method according to claim 20 incorporating the features of the microbe as defined in any one of claims 2 to 12.

## Patentansprüche

1. Zusammensetzung, umfassend eine pathogene Mikrobe mit einer attenuierenden Mutation in einem chromosomalen Gen, wobei die Mutation eine einen rekombinanten Transkriptionsterminator enthaltende Insertionssequenz umfasst, in einer pharmazeutisch verträglichen Zubereitung.

2. Zusammensetzung nach Anspruch 1 ferner umfassend ein rekombinantes, ein gewünschtes Genprodukt codierendes Gen.

3. Zusammensetzung nach Anspruch 1 oder 2, wobei der Transkriptionsterminator rrnB 5S rRNA T1T2, trpA, T4 Gen 32, T4 ipIII Gen oder rrfG 5S rRNA ist.

4. Zusammensetzung nach einem der Ansprüche 1 bis 3, wobei das chromosomale Gen ein pab Gen, ein pur Gen, ein aro Gen, asd, ein dap Gen, nadA, pncB, galE, pmi, fur, rpsL, ompR, htrA, hemA, cdt, cya, crp, phoP, phoQ, rfc, poxR oder galU ist.

5. Zusammensetzung nach einem der Ansprüche 1 bis 4, wobei die Mikrobe eine Salmonella Mikrobe, Shigella Mikrobe, Escherichia Mikrobe oder ein Hybrid davon ist.

6. Zusammensetzung nach einem der Ansprüche 1 bis 5, wobei der Transkriptionsterminator in dem phoP Gen insertiert vorliegt.

7. Zusammensetzung nach einem der Ansprüche 1 bis 6 ferner umfassend eine Deletionsmutation in dem codierenden Bereich des Gens, in dem Promotorbereich des Gens oder in dem codierenden Bereich und dem Promotorbereich des Gens.

8. Zusammensetzung nach einem der Ansprüche 2 bis 7, wobei das rekombinante Gen für ein Genprodukt eines Pathogens codiert.

9. Zusammensetzung nach Anspruch 8, wobei das Pathogen ein Virus, ein Bakterium, ein Protozoon, ein Parasit oder ein Pilz ist.

10. Zusammensetzung nach einem der Ansprüche 2 bis 9, wobei das rekombinante Gen für ein Produkt codiert, das zur Suppression, Modulation oder Verstärkung einer Immunantwort in einem Individuum fähig ist.

11. Zusammensetzung nach einem der Ansprüche 2 bis 10, wobei das rekombinante Gen für ein Autoantigen oder ein Allergen eines Individuums codiert.

12. Zusammensetzung nach Anspruch 11, wobei das Autoantigen ein gametspezifisches Antigen ist.

13. Vakzine, umfassend die Mikrobe nach einem der Ansprüche 1 bis 12.

14. Vakzine nach Anspruch 13, wobei die Insertionssequenz keinen rekombinanten Promotor enthält.

15. Verwendung einer pathogenen Mikrobe mit einer attenuierenden Mutation, umfassend eine einen rekombinanten Transkriptionsterminator enthaltende Insertionssequenz, in einem chromosomalen Gen und einem rekombinanten, für das gewünschte Genprodukt codierenden Gen zur Herstellung eines Medikaments zur Verabreichung eines gewünschten Genproduktes an ein Individuum.

16. Verwendung nach Anspruch 15, einschließlich der in einem der Ansprüche 2 bis 12 und 14 definierten Merkmale der Mikrobe.

17. Verwendung einer pathogenen Mikrobe mit einer attenuierenden Mutation, umfassend eine einen rekombinanten Transkriptionsterminator enthaltende Insertionssequenz, in einem chromosomalen Gen zur Herstellung eines Medikaments zur Immunisierung eines Individuums gegen ein Pathogen.

18. Verwendung nach Anspruch 17, einschließlich der in einem der Ansprüche 2 bis 12 definierten Merkmale der Mikrobe.

19. Verwendung nach Anspruch 18, wobei die Mikrobe ferner ein rekombinantes, für ein Epitop des Pathogen codierendes Gen umfasst.

20. Verfahren zur Herstellung einer Träger-Mikrobe zur Verabreichung eines gewünschten Genprodukts an ein Individuum, umfassend das Erzeugen einer pathogenen Mikrobe mit einem rekombinanten, für das gewünschte Genprodukt codierenden Gen und einer attenuierenden Mutation, umfassend eine einen Transkriptionsterminator enthaltende Insertionssequenz, in einem chromosomalen Gen.

21. Verfahren nach Anspruch 20, einschließlich der in einem der Ansprüche 2 bis 12 definierten Merkmale der Mikrobe.

## Revendications

1. Composition comprenant un microbe pathogène ayant une mutation atténuante dans un gène chromosomique, ladite mutation comprenant une séquence d'insertion qui contient un signal de terminaison de la transcription recombinant, dans une préparation pharmaceutiquement acceptable.

2. Composition selon la revendication 1, comprenant en outre un gène recombinant codant pour un produit de gène désiré.

3. Composition selon la revendication 1 ou 2, dans laquelle le signal de terminaison de la transcription est rrnB 5s rRNA T1T2, trpA, le gène T4 32, le gène T4 ipIII ou rrfG 5 S rRNA.

4. Composition selon l'une quelconque des revendications 1 à 3, dans laquelle le gène chromosomique est un gène pab, un gène pur, un gène aro, asd, un gène dap, nadA, pncB, gale, pmi, fur, rpsL, ompR, htrA, hemA, cdt, cya, crp, phoP, phoQ, rfc, poxR ou galU.

5. Composition selon l'une quelconque des revendications 1 à 4, dans laquelle le microbe est un *Salmonella*, *Shigella* ou *Escherichia* ou un hybride de ceux-ci.

6. Composition selon l'une quelconque des revendications 1 à 5, dans laquelle le signal de terminaison de la transcription est inséré dans le gène phoP.

7. Composition selon l'une quelconque des revendications 1 à 6, comprenant en outre une mutation par délétion dans la région de codage dudit gène, dans la région du promoteur dudit gène ou à la fois dans la région de codage et la région du promoteur dudit gène.

8. Composition selon l'une quelconque des revendications 2 à 7, dans laquelle le gène recombinant code pour un produit de gène d'un pathogène.

9. Composition selon la revendication 8, dans laquelle le pathogène est un virus, une bactérie, un protozoaire, un parasite ou un champignon.

10. Composition selon l'une quelconque des revendications 2 à 9, dans laquelle le gène recombinant code pour un produit capable de supprimer, moduler ou augmenter une réponse immunitaire chez l'individu.

11. Composition selon l'une quelconque des revendications 2 à 10, dans laquelle le gène recombinant code pour un auto-antigène ou un allergène pour l'individu.

12. Composition selon la revendication 11, dans laquelle l'auto-antigène est un antigène spécifique d'un gamète.

13. Vaccin incorporant le microbe tel que défini dans l'une quelconque des revendications 1 à 12.

14. Vaccin selon la revendication 13, dans lequel la séquence d'insertion ne contient pas de promoteur recombinant.

15. Utilisation d'un microbe pathogène ayant une mutation atténuante comprenant une séquence d'insertion contenant un signal de terminaison de la transcription recombinant dans un gène chromosomique et un gène recombinant codant pour le produit de gène désiré dans la fabrication d'un médicament pour la délivrance d'un produit de gène désiré à un individu.

16. Utilisation selon la revendication 15, incorporant les caractéristiques du microbe telles que définies dans l'une quelconque des revendications 2 à 12 et 14.

17. Utilisation d'un microbe pathogène ayant une mutation atténuante comprenant une séquence d'insertion contenant un signal de terminaison de la transcription recombinant dans un gène chromosomique dans la fabrication d'un médicament pour immuniser un individu contre un pathogène.

18. Utilisation selon la revendication 17, incorporant les caractéristiques du microbe telles que définies dans l'une quelconque des revendications 2 à 12.

19. Utilisation selon la revendication 18, où le microbe comprend en outre un gène recombinant codant pour un épitope du pathogène.

20. Méthode pour la production d'un microbe support pour la délivrance d'un produit de gène désiré à un individu, la méthode comprenant la génération d'un microbe pathogène ayant un gène recombinant codant pour le produit de gène désiré et une mutation atténuante comprenant une séquence d'insertion qui contient un signal de terminaison de la transcription dans un gène chromosomique.

21. Méthode selon la revendication 20, incorporant les caractéristiques du microbe telles que définies dans l'une quelconque des revendications 2 à 12.
